# EUROPEAN PATENT APPLICATION

(11) **EP 1 637 595 A1**
(43) Date of publication of application: **22.03.2006**
(21) Application number: 04447202.5
(22) Date of filing: 16.09.2004
(51) Int. Cl.: C12N 9/02, C12N 15/53

(54) **Overexpressed and purified Aspergillus ficuum oxidase and nucleic acid encoding same**

(71) Applicant: PURATOS N.V., 1702 Groot-Bijgaarden (BE)
(72) Inventor: Arnaut, Filip, 1761 Roosdaal (BE); Dauvrin, Thierry, 4218 Couthuin (BE); Contreras, Roland, 9820 Merelbeke (BE); Vanneste, Guy, 1348 Louvain-la-Neuve (BE); Viaene, Jasmine, 8490 Varsenare (BE)
(74) Representative: Van Malderen, Joelle

(57) **Abstract**

The present invention relates to a new isolated nucleic acid sequence comprising a gene that encodes a new fungal oxidase enzyme, and nucleic acid fragments thereof.

It also relates to said new fungal oxidase isolated and purified from *Aspergillus ficuum,* its amino acid sequence as shown in SEQ ID NO 40 or 41, and functional equivalents or derivatives thereof.

The present invention also relates to constructs, vectors and hosts cells comprising a nucleic acid molecule of the invention, as well as methods for producing an oxidase of the invention.

The present invention also relates to the use of an oxidase of the invention in industrial processes.

SEQUENCE LISTING

<110>  PURATOS N.V.

<120>  Overexpressed and purified Aspergillus ficuum oxidase and nucleic
       acid encoding the same

<130>  BP.PURA.030A/EP

<160>  52

<170>  PatentIn version 3.3

<210>  1
<211>  2487
<212>  DNA
<213>  Aspergillus ficuum

<400>  1
taattgaagc ttgcggtgag caagacagtc tgcaccctgc tagaaatgtt cgtatgccgg      60

taggccctgc atatgacgga tccagtatct tggccatcgg acggcattat gacaagccct     120

agtcatgaac ctttcgctat acgtctcggg tcctccatcc cttgccttga ccttcgggca     180

tcaatggcat caggtcaatg tgcctcttga gttctgtcat tataaaagcc tcaaacttct     240

cgcaggatga agggaactca accacgacaa accagtccgc gactcggccc tgttcgactg     300

tatatattct gtggaactag gtagttggtt ccttgtatat ttctttattt ggttttttga      360

gccccagcat gagtatatct caaagcaggc tggccctgtt ggccctttgc ttcgtgcaat     420

gggtgtccgg ccgtgttgtg cagttccagc tagacctgac ctatgaagat gtcagtgtcg     480

ctggtgatgt acacaaggct attgtgagta atggacagat tcctgggcct acgttgtggt     540

tgaagcaggg tgatgatgtc gaatttttgg tcaacaactc gatgtcaatc aatactacgg     600

ttcattttca tggtgagtac tacctctcga ttgtatgatc ctttggcttc gatgttcaga     660

acgccttttc ctatagagct ttactaaaga tttgtaggta tcgagcaact aggcaccccg     720

tggtcagatg gtgtacctgg actctcgcag gagcagatca agccgggcga acagtttctc     780

tataaatgga aggcaagcca gtatggctcg tacatttatc actcgcacac acgggctcag     840

attgatgatg gcctctatgg ggctatctac attgaaccgg cagactccgt ggagaggcct     900

ttccatttga tcgctggatc tgatgcggat gagcagcagg cgatgttggc agctgaaaag     960

aatacacggc cagttctcat ctcggattgg cgtgcctttt cctcgcatga tattctccag    1020

attcaaaccg aatctggtgt tgaggcctac tgtgccaact ctgttttgat caacggaaag    1080

ggctcggtga tttgcccgtc tcaggaacac atcaatgctg ccacgacacc gcagcaaaag    1140

cagatcctgg gcaacttgac tctgactgat atggggtatg aagtcccaag acttgatgac    1200

ggttacaatg ctgatcctgg ctagatgcct gcctcccacg ccgggtgttg tgggtccgta    1260

cccgtacgac ctcagcaaga ttcccaaagg tttctacgaa ggatgcacac ctagtgaagg    1320

gcccacagag gttttcaagg tcaactcctc atcgcggtat gctagctttg actttattag    1380

tatggctggc tcgacttcgc ttgtgttctc catcgatgag catccgatgt atgtttacgc    1440

ggtcgatggc cgttatgttg aacctttgct tgtcgaagct gttactgtgc ctgtcgcttc    1500

aagatactca gttatggtgc cactcaagtc ggaagaccag gcaggagatt atactatccg 1560
cgttgccaat aactatgcga accagctcat caacgggacc gccgtcctgt cgtatgacac 1620
cgctacaccg aagcagatcg gtacctcgca gccttatatc aacgaggctg gcgctaatgc 1680
cactgcctcc acggtcattt tgaacgaaac cgacgtgata cccttccccg tggtagcacc 1740
tgctaagaag gcggaccgga cttacatcct gaatgtcgaa aatgccaatt catcctacac 1800
ctggaccctg ggcaaccagt accccgtgtc gaatgaagag cttcgccctc ctgtcttgtt 1860
caacctcagc tcaatctcgc aggcgtactc ggccatgacg gagtacggta cctgggttga 1920
tctcatcatc aacatcacca cctcgggcca gcctcaacac cccatccaca agcactccaa 1980
caagtacttc gtgatcggaa ccggtaacca gcccttcatc tggtcctccg tggaagaggc 2040
aatgaaagac ataccggaga atttcaactt cgaaaacccc cagatgcggg acactttcta 2100
ctcgccaagc tcaagcacag ggccgagctg gctggcaatg cgctaccata tcgtgaaccc 2160
agggccattc ctacttcatt gccatcttca gatgcaccat gtcggtgggt tggcactggc 2220
cttgctggac ggggtggacg cctggccaac cgacattccc gagggatatc ggttgccagt 2280
gatgccaatc tagggacttt gactcaattg tacagaggtg gacgagagta tctacttgta 2340
atatggccat atagcgcctc tcatatagat ccggtgtaat attatttatt tactacataa 2400
tgaacatgct tggtgactta ccaattcccc agttatggct ccaaagtaaa agtgagagca 2460
atcacaccaa atcaggcaca gcagtaa 2487

<210> 2
<211> 1791
<212> DNA
<213> Aspergillus ficuum

<400> 2
atgagtatat ctcaaagcag gctggccctg ttggcccttt gcttcgtgca atgggtgtcc 60
ggccgtgttg tgcagttcca gctagacctg acctatgaag atgtcagtgt cgctggtgat 120
gtacacaagg ctattgtgag taatggacag attcctgggc ctacgttgtg gttgaagcag 180
ggtgatgatg tcgaattttt ggtcaacaac tcgatgtcaa tcaatactac ggttcatttt 240
catggtatcg agcaactagg caccccgtgg tcagatggtg tacctggact ctcgcaggag 300
cagatcaagc cgggcgaaca gtttctctat aaatggaagg caagccagta tggctcgtac 360
atttatcact cgcacacacg ggctcagatt gatgatggcc tctatggggc tatctacatt 420
gaaccggcag actccgtgga gaggcctttc catttgatcg ctggatctga tgcggatgag 480
cagcaggcga tgttggcagc tgaaaagaat acacggccag ttctcatctc ggattggcgt 540
gccttttcct cgcatgatat tctccagatt caaaccgaat ctggtgttga ggcctactgt 600
gccaactctg ttttgatcaa cggaaagggc tcggtgattt gcccgtctca ggaacacatc 660
aatgctgcca cgacaccgca gcaaaagcag atcctgggca acttgactct gactgatatg 720
ggatgcctgc ctcccacgcc gggtgttgtg ggtccgtacc cgtacgacct cagcaagatt 780
cccaaaggtt tctacgaagg atgcacacct agtgaagggc ccacagaggt tttcaaggtc 840

aactcctcat cgcggtatgc tagctttgac tttattagta tggctggctc gacttcgctt 900

gtgttctcca tcgatgagca tccgatgtat gtttacgcgg tcgatggccg ttatgttgaa 960

cctttgcttg tcgaagctgt tactgtgcct gtcgcttcaa gatactcagt tatggtgcca 1020

ctcaagtcgg aagaccaggc aggagattat actatccgcg ttgccaataa ctatgcgaac 1080

cagctcatca acgggaccgc cgtcctgtcg tatgacaccg ctacaccgaa gcagatcggt 1140

acctcgcagc cttatatcaa cgaggctggc gctaatgcca ctgcctccac ggtcattttg 1200

aacgaaaccg acgtgatacc cttccccgtg gtagcacctg ctaagaaggc ggaccggact 1260

tacatcctga atgtcgaaaa tgccaattca tcctacacct ggaccctggg caaccagtac 1320

cccgtgtcga atgaagagct tcgccctcct gtcttgttca acctcagctc aatctcgcag 1380

gcgtactcgg ccatgacgga gtacggtacc tgggttgatc tcatcatcaa catcaccacc 1440

tcgggccagc ctcaacaccc catccacaag cactccaaca agtacttcgt gatcggaacc 1500

ggtaaccagc ccttcatctg gtcctccgtg gaagaggcaa tgaaagacat accggagaat 1560

ttcaacttcg aaaaccccca gatgcgggac actttctact cgccaagctc aagcacaggg 1620

ccgagctggc tggcaatgcg ctaccatatc gtgaacccag ggccattcct acttcattgc 1680

catcttcaga tgcaccatgt cggtgggttg gcactggcct tgctggacgg ggtggacgcc 1740

tggccaaccg acattcccga gggatatcgg ttgccagtga tgccaatcta g 1791

<210> 3
<211> 1925
<212> DNA
<213> Aspergillus ficuum

<400> 3
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtagg tatcgagcaa ctaggcaccc cgtggtcaga 360

tggtgtacct ggactctcgc aggagcagat caagccgggc gaacagtttc tctataaatg 420

gaaggcaagc cagtatggct cgtacattta tcactcgcac acacgggctc agattgatga 480

tggcctctat ggggctatct acattgaacc ggcagactcc gtggagaggc ctttccattt 540

gatcgctgga tctgatgcgg atgagcagca ggcgatgttg gcagctgaaa agaatacacg 600

gccagttctc atctcggatt ggcgtgcctt ttcctcgcat gatattctcc agattcaaac 660

cgaatctggt gttgaggcct actgtgccaa ctctgttttg atcaacggaa agggctcggt 720

gatttgcccg tctcaggaac acatcaatgc tgccacgaca ccgcagcaaa agcagatcct 780

gggcaacttg actctgactg atatggggta tgaagtccca agacttgatg acggttacaa 840

tgctgatcct ggctagatgc ctgcctccca cgccgggtgt tgtgggtccg tacccgtacg 900

acctcagcaa gattcccaaa ggtttctacg aaggatgcac acctagtgaa gggcccacag 960

aggttttcaa ggtcaactcc tcatcgcggt atgctagctt tgactttatt agtatggctg 1020

gctcgacttc gcttgtgttc tccatcgatg agcatccgat gtatgtttac gcggtcgatg 1080

gccgttatgt tgaacctttg cttgtcgaag ctgttactgt gcctgtcgct tcaagatact 1140

cagttatggt gccactcaag tcggaagacc aggcaggaga ttatactatc cgcgttgcca 1200

ataactatgc gaaccagctc atcaacggga ccgccgtcct gtcgtatgac accgctacac 1260

cgaagcagat cggtacctcg cagccttata tcaacgaggc tggcgctaat gccactgcct 1320

ccacggtcat tttgaacgaa accgacgtga tacccttccc cgtggtagca cctgctaaga 1380

aggcggaccg gacttacatc ctgaatgtcg aaaatgccaa ttcatcctac acctggaccc 1440

tgggcaacca gtaccccgtg tcgaatgaag agcttcgccc tcctgtcttg ttcaacctca 1500

gctcaatctc gcaggcgtac tcggccatga cggagtacgg tacctgggtt gatctcatca 1560

tcaacatcac cacctcgggc cagcctcaac accccatcca caagcactcc aacaagtact 1620

tcgtgatcgg aaccggtaac cagcccttca tctggtcctc cgtggaagag gcaatgaaag 1680

acataccgga gaatttcaac ttcgaaaacc cccagatgcg ggacactttc tactcgccaa 1740

gctcaagcac agggccgagc tggctggcaa tgcgctacca tatcgtgaac ccagggccat 1800

tcctacttca ttgccatctt cagatgcacc atgtcggtgg gttggcactg gccttgctgg 1860

acggggtgga cgcctggcca accgacattc ccgagggata tcggttgcca gtgatgccaa 1920

tctag 1925

<210> 4
<211> 1876
<212> DNA
<213> Aspergillus ficuum

<400> 4
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtagg tatcgagcaa ctaggcaccc cgtggtcaga 360

tggtgtacct ggactctcgc aggagcagat caagccgggc gaacagtttc tctataaatg 420

gaaggcaagc cagtatggct cgtacattta tcactcgcac acacgggctc agattgatga 480

tggcctctat ggggctatct acattgaacc ggcagactcc gtggagaggc ctttccattt 540

gatcgctgga tctgatgcgg atgagcagca ggcgatgttg gcagctgaaa agaatacacg 600

gccagttctc atctcggatt ggcgtgcctt ttcctcgcat gatattctcc agattcaaac 660

cgaatctggt gttgaggcct actgtgccaa ctctgttttg atcaacggaa agggctcggt 720

gatttgcccg tctcaggaac acatcaatgc tgccacgaca ccgcagcaaa agcagatcct 780

gggcaacttg actctgactg atatgggatg cctgcctccc acgccgggtg ttgtgggtcc 840
gtacccgtac gacctcagca agattcccaa aggtttctac gaaggatgca cacctagtga 900
agggcccaca gaggttttca aggtcaactc ctcatcgcgg tatgctagct ttgactttat 960
tagtatggct ggctcgactt cgcttgtgtt ctccatcgat gagcatccga tgtatgttta 1020
cgcggtcgat ggccgttatg ttgaaccttt gcttgtcgaa gctgttactg tgcctgtcgc 1080
ttcaagatac tcagttatgg tgccactcaa gtcggaagac caggcaggag attatactat 1140
ccgcgttgcc aataactatg cgaaccagct catcaacggg accgccgtcc tgtcgtatga 1200
caccgctaca ccgaagcaga tcggtacctc gcagccttat atcaacgagg ctggcgctaa 1260
tgccactgcc tccacggtca ttttgaacga aaccgacgtg atacccttcc ccgtggtagc 1320
acctgctaag aaggcggacc ggacttacat cctgaatgtc gaaaatgcca attcatccta 1380
cacctggacc ctgggcaacc agtaccccgt gtcgaatgaa gagcttcgcc ctcctgtctt 1440
gttcaacctc agctcaatct cgcaggcgta ctcggccatg acggagtacg gtacctgggt 1500
tgatctcatc atcaacatca ccacctcggg ccagcctcaa caccccatcc acaagcactc 1560
caacaagtac ttcgtgatcg gaaccggtaa ccagcccttc atctggtcct ccgtggaaga 1620
ggcaatgaaa gacataccgg agaatttcaa cttcgaaaac ccccagatgc gggacacttt 1680
ctactcgcca agctcaagca cagggccgag ctggctggca atgcgctacc atatcgtgaa 1740
cccagggcca ttcctacttc attgccatct tcagatgcac catgtcggtg ggttggcact 1800
ggccttgctg gacggggtgg acgcctggcc aaccgacatt cccgagggat atcggttgcc 1860
agtgatgcca atctag 1876

<210> 5
<211> 1840
<212> DNA
<213> Aspergillus ficuum

<400> 5
atgagtatat ctcaaagcag gctggccctg ttggcccttt gcttcgtgca atgggtgtcc 60
ggccgtgttg tgcagttcca gctagacctg acctatgaag atgtcagtgt cgctggtgat 120
gtacacaagg ctattgtgag taatggacag attcctgggc ctacgttgtg gttgaagcag 180
ggtgatgatg tcgaattttt ggtcaacaac tcgatgtcaa tcaatactac ggttcatttt 240
catggtatcg agcaactagg caccccgtgg tcagatggtg tacctggact ctcgcaggag 300
cagatcaagc cgggcgaaca gtttctctat aaatggaagg caagccagta tggctcgtac 360
atttatcact cgcacacacg ggctcagatt gatgatggcc tctatggggc tatctacatt 420
gaaccggcag actccgtgga gaggcctttc catttgatcg ctggatctga tgcggatgag 480
cagcaggcga tgttggcagc tgaaaagaat acacggccag ttctcatctc ggattggcgt 540
gccttttcct cgcatgatat tctccagatt caaaccgaat ctggtgttga ggcctactgt 600
gccaactctg ttttgatcaa cggaaagggc tcggtgattt gcccgtctca ggaacacatc 660
aatgctgcca cgacaccgca gcaaaagcag atcctgggca acttgactct gactgatatg 720

gggtatgaag tcccaagact tgatgacggt tacaatgctg atcctggcta gatgcctgcc 780

tcccacgccg ggtgttgtgg gtccgtaccc gtacgacctc agcaagattc ccaaaggttt 840

ctacgaagga tgcacaccta gtgaagggcc cacagaggtt ttcaaggtca actcctcatc 900

gcggtatgct agctttgact ttattagtat ggctggctcg acttcgcttg tgttctccat 960

cgatgagcat ccgatgtatg tttacgcggt cgatggccgt tatgttgaac ctttgcttgt 1020

cgaagctgtt actgtgcctg tcgcttcaag atactcagtt atggtgccac tcaagtcgga 1080

agaccaggca ggagattata ctatccgcgt tgccaataac tatgcgaacc agctcatcaa 1140

cgggaccgcc gtcctgtcgt atgacaccgc tacaccgaag cagatcggta cctcgcagcc 1200

ttatatcaac gaggctggcg ctaatgccac tgcctccacg gtcattttga acgaaaccga 1260

cgtgataccc ttccccgtgg tagcacctgc taagaaggcg gaccggactt acatcctgaa 1320

tgtcgaaaat gccaattcat cctacacctg gaccctgggc aaccagtacc ccgtgtcgaa 1380

tgaagagctt cgccctcctg tcttgttcaa cctcagctca atctcgcagg cgtactcggc 1440

catgacggag tacggtacct gggttgatct catcatcaac atcaccacct cgggccagcc 1500

tcaacacccc atccacaagc actccaacaa gtacttcgtg atcggaaccg gtaaccagcc 1560

cttcatctgg tcctccgtgg aagaggcaat gaaagacata ccggagaatt tcaacttcga 1620

aaacccccag atgcgggaca ctttctactc gccaagctca agcacagggc cgagctggct 1680

ggcaatgcgc taccatatcg tgaacccagg gccattccta cttcattgcc atcttcagat 1740

gcaccatgtc ggtgggttgg cactggcctt gctggacggg gtggacgcct ggccaaccga 1800

cattcccgag ggatatcggt tgccagtgat gccaatctag 1840

<210> 6
<211> 2293
<212> DNA
<213> Aspergillus ficuum

<400> 6
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggttttttga 360

gccccagcat gagtatatct caaagcaggc tggccctgtt ggccctttgc ttcgtgcaat 420

gggtgtccgg ccgtgttgtg cagttccagc tagacctgac ctatgaagat gtcagtgtcg 480

ctggtgatgt acacaaggct attgtgagta atggacagat tcctgggcct acgttgtggt 540

tgaagcaggg tgatgatgtc gaatttttgg tcaacaactc gatgtcaatc aatactacgg 600

ttcattttca tggtgagtac tacctctcga ttgtatgatc ctttggcttc gatgttcaga 660

acgccttttc ctatagagct ttactaaaga tttgtaggta tcgagcaact aggcaccccg 720

tggtcagatg gtgtacctgg actctcgcag gagcagatca agccgggcga acagtttctc 780
tataaatgga aggcaagcca gtatggctcg tacatttatc actcgcacac acgggctcag 840
attgatgatg gcctctatgg ggctatctac attgaaccgg cagactccgt ggagaggcct 900
ttccatttga tcgctggatc tgatgcggat gagcagcagg cgatgttggc agctgaaaag 960
aatacacggc cagttctcat ctcggattgg cgtgcctttt cctcgcatga tattctccag 1020
attcaaaccg aatctggtgt tgaggcctac tgtgccaact ctgttttgat caacggaaag 1080
ggctcggtga tttgcccgtc tcaggaacac atcaatgctg ccacgacacc gcagcaaaag 1140
cagatcctgg gcaacttgac tctgactgat atggggtatg aagtcccaag acttgatgac 1200
ggttacaatg ctgatcctgg ctagatgcct gcctcccacg ccgggtgttg tgggtccgta 1260
cccgtacgac ctcagcaaga ttcccaaagg tttctacgaa ggatgcacac ctagtgaagg 1320
gcccacagag gttttcaagg tcaactcctc atcgcggtat gctagctttg actttattag 1380
tatggctggc tcgacttcgc ttgtgttctc catcgatgag catccgatgt atgtttacgc 1440
ggtcgatggc cgttatgttg aacctttgct tgtcgaagct gttactgtgc ctgtcgcttc 1500
aagatactca gttatggtgc cactcaagtc ggaagaccag gcaggagatt atactatccg 1560
cgttgccaat aactatgcga accagctcat caacggaccc gccgtcctgt cgtatgacac 1620
cgctacaccg aagcagatcg gtacctcgca gccttatatc aacgaggctg gcgctaatgc 1680
cactgcctcc acggtcattt tgaacgaaac cgacgtgata cccttccccg tggtagcacc 1740
tgctaagaag gcggaccgga cttacatcct gaatgtcgaa aatgccaatt catcctacac 1800
ctggaccctg ggcaaccagt accccgtgtc gaatgaagag cttcgccctc ctgtcttgtt 1860
caacctcagc tcaatctcgc aggcgtactc ggccatgacg gagtacggta cctgggttga 1920
tctcatcatc aacatcacca cctcgggcca gcctcaacac cccatccaca agcactccaa 1980
caagtacttc gtgatcggaa ccggtaacca gcccttcatc tggtcctccg tggaagaggc 2040
aatgaaagac ataccggaga atttcaactt cgaaaacccc cagatgcggg acactttcta 2100
ctcgccaagc tcaagcacag ggccgagctg gctggcaatg cgctaccata tcgtgaaccc 2160
agggccattc ctacttcatt gccatcttca gatgcaccat gtcggtgggt tggcactggc 2220
cttgctggac ggggtggacg cctggccaac cgacattccc gagggatatc ggttgccagt 2280
gatgccaatc tag 2293

<210> 7
<211> 2159
<212> DNA
<213> Aspergillus ficuum

<400> 7
taattgaagc ttgcggtgag caagacagtc tgcaccctgc tagaaatgtt cgtatgccgg 60
taggccctgc atatgacgga tccagtatct tggccatcgg acggcattat gacaagccct 120
agtcatgaac ctttcgctat acgtctcggg tcctccatcc cttgccttga ccttcgggca 180
tcaatggcat caggtcaatg tgcctcttga gttctgtcat tataaaagcc tcaaacttct 240

cgcaggatga agggaactca accacgacaa accagtccgc gactcggccc tgttcgactg 300
tatatattct gtggaactag gtagttggtt ccttgtatat ttctttattt ggttttttga 360
gccccagcat gagtatatct caaagcaggc tggccctgtt ggccctttgc ttcgtgcaat 420
gggtgtccgg ccgtgttgtg cagttccagc tagacctgac ctatgaagat gtcagtgtcg 480
ctggtgatgt acacaaggct attgtgagta atggacagat tcctgggcct acgttgtggt 540
tgaagcaggg tgatgatgtc gaatttttgg tcaacaactc gatgtcaatc aatactacgg 600
ttcattttca tggtatcgag caactaggca ccccgtggtc agatggtgta cctggactct 660
cgcaggagca gatcaagccg ggcgaacagt ttctctataa atggaaggca agccagtatg 720
gctcgtacat ttatcactcg cacacacggg ctcagattga tgatggcctc tatggggcta 780
tctacattga accggcagac tccgtggaga ggcctttcca tttgatcgct ggatctgatg 840
cggatgagca gcaggcgatg ttggcagctg aaaagaatac acggccagtt ctcatctcgg 900
attggcgtgc cttttcctcg catgatattc tccagattca aaccgaatct ggtgttgagg 960
cctactgtgc caactctgtt ttgatcaacg gaaagggctc ggtgatttgc ccgtctcagg 1020
aacacatcaa tgctgccacg acaccgcagc aaaagcagat cctgggcaac ttgactctga 1080
ctgatatggg atgcctgcct cccacgccgg gtgttgtggg tccgtacccg tacgacctca 1140
gcaagattcc caaaggtttc tacgaaggat gcacacctag tgaagggccc acagaggttt 1200
tcaaggtcaa ctcctcatcg cggtatgcta gctttgactt tattagtatg gctggctcga 1260
cttcgcttgt gttctccatc gatgagcatc cgatgtatgt ttacgcggtc gatggccgtt 1320
atgttgaacc tttgcttgtc gaagctgtta ctgtgcctgt cgcttcaaga tactcagtta 1380
tggtgccact caagtcggaa gaccaggcag gagattatac tatccgcgtt gccaataact 1440
atgcgaacca gctcatcaac gggaccgccg tcctgtcgta tgacaccgct acaccgaagc 1500
agatcggtac ctcgcagcct tatatcaacg aggctggcgc taatgccact gcctccacgg 1560
tcattttgaa cgaaaccgac gtgataccct tccccgtggt agcacctgct aagaaggcgg 1620
accggactta catcctgaat gtcgaaaatg ccaattcatc ctacacctgg accctgggca 1680
accagtaccc cgtgtcgaat gaagagcttc gccctcctgt cttgttcaac ctcagctcaa 1740
tctcgcaggc gtactcggcc atgacggagt acggtacctg ggttgatctc atcatcaaca 1800
tcaccacctc gggccagcct caacacccca tccacaagca ctccaacaag tacttcgtga 1860
tcggaaccgg taaccagccc ttcatctggt cctccgtgga agaggcaatg aaagacatac 1920
cggagaattt caacttcgaa aacccccaga tgcgggacac tttctactcg ccaagctcaa 1980
gcacagggcc gagctggctg gcaatgcgct accatatcgt gaacccaggg ccattcctac 2040
ttcattgcca tcttcagatg caccatgtcg gtgggttggc actggccttg ctggacgggg 2100
tggacgcctg gccaaccgac attcccgagg gatatcggtt gccagtgatg ccaatctag 2159

<210> 8
<211> 1985
<212> DNA
<213> Aspergillus ficuum

<400> 8
atgagtatat ctcaaagcag gctggccctg ttggcccttt gcttcgtgca atgggtgtcc 60
ggccgtgttg tgcagttcca gctagacctg acctatgaag atgtcagtgt cgctggtgat 120
gtacacaagg ctattgtgag taatggacag attcctgggc ctacgttgtg gttgaagcag 180
ggtgatgatg tcgaattttt ggtcaacaac tcgatgtcaa tcaatactac ggttcatttt 240
catggtatcg agcaactagg caccccgtgg tcagatggtg tacctggact ctcgcaggag 300
cagatcaagc cgggcgaaca gtttctctat aaatggaagg caagccagta tggctcgtac 360
atttatcact cgcacacacg ggctcagatt gatgatggcc tctatggggc tatctacatt 420
gaaccggcag actccgtgga gaggcctttc catttgatcg ctggatctga tgcggatgag 480
cagcaggcga tgttggcagc tgaaaagaat acacggccag ttctcatctc ggattggcgt 540
gccttttcct cgcatgatat tctccagatt caaaccgaat ctggtgttga ggcctactgt 600
gccaactctg ttttgatcaa cggaaagggc tcggtgattt gcccgtctca ggaacacatc 660
aatgctgcca cgacaccgca gcaaaagcag atcctgggca acttgactct gactgatatg 720
ggatgcctgc ctcccacgcc gggtgttgtg ggtccgtacc cgtacgacct cagcaagatt 780
cccaaaggtt tctacgaagg atgcacacct agtgaagggc ccacagaggt tttcaaggtc 840
aactcctcat cgcggtatgc tagctttgac tttattagta tggctggctc gacttcgctt 900
gtgttctcca tcgatgagca tccgatgtat gtttacgcgg tcgatggccg ttatgttgaa 960
cctttgcttg tcgaagctgt tactgtgcct gtcgcttcaa gatactcagt tatggtgcca 1020
ctcaagtcgg aagaccaggc aggagattat actatccgcg ttgccaataa ctatgcgaac 1080
cagctcatca acgggaccgc cgtcctgtcg tatgacaccg ctacaccgaa gcagatcggt 1140
acctcgcagc cttatatcaa cgaggctggc gctaatgcca ctgcctccac ggtcattttg 1200
aacgaaaccg acgtgatacc cttccccgtg gtagcacctg ctaagaaggc ggaccggact 1260
tacatcctga atgtcgaaaa tgccaattca tcctacacct ggaccctggg caaccagtac 1320
cccgtgtcga atgaagagct tcgccctcct gtcttgttca acctcagctc aatctcgcag 1380
gcgtactcgg ccatgacgga gtacggtacc tgggttgatc tcatcatcaa catcaccacc 1440
tcgggccagc ctcaacaccc catccacaag cactccaaca agtacttcgt gatcggaacc 1500
ggtaaccagc ccttcatctg gtcctccgtg gaagaggcaa tgaaagacat accggagaat 1560
ttcaacttcg aaaaccccca gatgcgggac actttctact cgccaagctc aagcacaggg 1620
ccgagctggc tggcaatgcg ctaccatatc gtgaacccag ggccattcct acttcattgc 1680
catcttcaga tgcaccatgt cggtgggttg gcactggcct tgctggacgg ggtggacgcc 1740
tggccaaccg acattcccga gggatatcgg ttgccagtga tgccaatcta gggactttga 1800
ctcaattgta cagaggtgga cgagagtatc tacttgtaat atggccatat agcgcctctc 1860
atatagatcc ggtgtaatat tatttattta ctacataatg aacatgcttg gtgacttacc 1920
aattccccag ttatggctcc aaagtaaaag tgagagcaat cacaccaaat caggcacagc 1980
agtaa 1985

<210> 9
<211> 2119
<212> DNA
<213> Aspergillus ficuum

<400> 9
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtagg tatcgagcaa ctaggcaccc cgtggtcaga 360

tggtgtacct ggactctcgc aggagcagat caagccgggc gaacagtttc tctataaatg 420

gaaggcaagc cagtatggct cgtacattta tcactcgcac acacgggctc agattgatga 480

tggcctctat ggggctatct acattgaacc ggcagactcc gtggagaggc ctttccattt 540

gatcgctgga tctgatgcgg atgagcagca ggcgatgttg gcagctgaaa agaatacacg 600

gccagttctc atctcggatt ggcgtgcctt ttcctcgcat gatattctcc agattcaaac 660

cgaatctggt gttgaggcct actgtgccaa ctctgttttg atcaacggaa agggctcggt 720

gatttgcccg tctcaggaac acatcaatgc tgccacgaca ccgcagcaaa agcagatcct 780

gggcaacttg actctgactg atatggggta tgaagtccca agacttgatg acggttacaa 840

tgctgatcct ggctagatgc ctgcctccca cgccgggtgt tgtgggtccg tacccgtacg 900

acctcagcaa gattcccaaa ggtttctacg aaggatgcac acctagtgaa gggcccacag 960

aggttttcaa ggtcaactcc tcatcgcggt atgctagctt tgactttatt agtatggctg 1020

gctcgacttc gcttgtgttc tccatcgatg agcatccgat gtatgtttac gcggtcgatg 1080

gccgttatgt tgaacctttg cttgtcgaag ctgttactgt gcctgtcgct tcaagatact 1140

cagttatggt gccactcaag tcggaagacc aggcaggaga ttatactatc cgcgttgcca 1200

ataactatgc gaaccagctc atcaacggga ccgccgtcct gtcgtatgac accgctacac 1260

cgaagcagat cggtacctcg cagccttata tcaacgaggc tggcgctaat gccactgcct 1320

ccacggtcat tttgaacgaa accgacgtga taccctttcc cgtggtagca cctgctaaga 1380

aggcggaccg gacttacatc ctgaatgtcg aaaatgccaa ttcatcctac acctggaccc 1440

tgggcaacca gtaccccgtg tcgaatgaag agcttcgccc tcctgtcttg ttcaacctca 1500

gctcaatctc gcaggcgtac tcggccatga cggagtacgg tacctgggtt gatctcatca 1560

tcaacatcac cacctcgggc cagcctcaac accccatcca caagcactcc aacaagtact 1620

tcgtgatcgg aaccggtaac cagcccttca tctggtcctc cgtggaagag gcaatgaaag 1680

acataccgga gaatttcaac ttcgaaaacc cccagatgcg ggacactttc tactcgccaa 1740

gctcaagcac agggccgagc tggctggcaa tgcgctacca tatcgtgaac ccagggccat 1800

tcctacttca ttgccatctt cagatgcacc atgtcggtgg gttggcactg gccttgctgg 1860

acggggtgga cgcctggcca accgacattc ccgagggata tcggttgcca gtgatgccaa 1920
tctagggact ttgactcaat tgtacagagg tggacgagag tatctacttg taatatggcc 1980
atatagcgcc tctcatatag atccggtgta atattattta tttactacat aatgaacatg 2040
cttggtgact taccaattcc ccagttatgg ctccaaagta aaagtgagag caatcacacc 2100
aaatcaggca cagcagtaa 2119

<210> 10
<211> 1985
<212> DNA
<213> Aspergillus ficuum

<400> 10
atgagtatat ctcaaagcag gctggccctg ttggcccttt gcttcgtgca atgggtgtcc 60
ggccgtgttg tgcagttcca gctagacctg acctatgaag atgtcagtgt cgctggtgat 120
gtacacaagg ctattgtgag taatggacag attcctgggc ctacgttgtg gttgaagcag 180
ggtgatgatg tcgaattttt ggtcaacaac tcgatgtcaa tcaatactac ggttcatttt 240
catggtatcg agcaactagg caccccgtgg tcagatggtg tacctggact ctcgcaggag 300
cagatcaagc cgggcgaaca gtttctctat aaatggaagg caagccagta tggctcgtac 360
atttatcact cgcacacacg ggctcagatt gatgatggcc tctatggggc tatctacatt 420
gaaccggcag actccgtgga gaggcctttc catttgatcg ctggatctga tgcggatgag 480
cagcaggcga tgttggcagc tgaaaagaat acacggccag ttctcatctc ggattggcgt 540
gccttttcct cgcatgatat tctccagatt caaaccgaat ctggtgttga ggcctactgt 600
gccaactctg ttttgatcaa cggaaagggc tcggtgattt gcccgtctca ggaacacatc 660
aatgctgcca cgacaccgca gcaaaagcag atcctgggca acttgactct gactgatatg 720
ggatgcctgc ctcccacgcc gggtgttgtg ggtccgtacc cgtacgacct cagcaagatt 780
cccaaaggtt tctacgaagg atgcacacct agtgaagggc ccacagaggt tttcaaggtc 840
aactcctcat cgcggtatgc tagctttgac tttattagta tggctggctc gacttcgctt 900
gtgttctcca tcgatgagca tccgatgtat gtttacgcgg tcgatggccg ttatgttgaa 960
cctttgcttg tcgaagctgt tactgtgcct gtcgcttcaa gatactcagt tatggtgcca 1020
ctcaagtcgg aagaccaggc aggagattat actatccgcg ttgccaataa ctatgcgaac 1080
cagctcatca acgggaccgc cgtcctgtcg tatgacaccg ctacaccgaa gcagatcggt 1140
acctcgcagc cttatatcaa cgaggctggc gctaatgcca ctgcctccac ggtcattttg 1200
aacgaaaccg acgtgatacc cttccccgtg gtagcacctg ctaagaaggc ggaccggact 1260
tacatcctga atgtcgaaaa tgccaattca tcctacacct ggaccctggg caaccagtac 1320
cccgtgtcga atgaagagct tcgccctcct gtcttgttca acctcagctc aatctcgcag 1380
gcgtactcgg ccatgacgga gtacggtacc tgggttgatc tcatcatcaa catcaccacc 1440
tcgggccagc ctcaacaccc catccacaag cactccaaca agtacttcgt gatcggaacc 1500
ggtaaccagc ccttcatctg gtcctccgtg gaagaggcaa tgaaagacat accggagaat 1560

ttcaacttcg aaaaccccca gatgcgggac actttctact cgccaagctc aagcacaggg 1620
ccgagctggc tggcaatgcg ctaccatatc gtgaacccag ggccattcct acttcattgc 1680
catcttcaga tgcaccatgt cggtgggttg gcactggcct tgctggacgg ggtggacgcc 1740
tggccaaccg acattcccga gggatatcgg ttgccagtga tgccaatcta gggactttga 1800
ctcaattgta cagaggtgga cgagagtatc tacttgtaat atggccatat agcgcctctc 1860
atatagatcc ggtgtaatat tatttattta ctacataatg aacatgcttg gtgacttacc 1920
aattccccag ttatggctcc aaagtaaaag tgagagcaat cacaccaaat caggcacagc 1980
agtaa 1985

<210> 11
<211> 1596
<212> DNA
<213> Aspergillus ficuum

<400> 11
gtatcgagca actaggcacc ccgtggtcag atggtgtacc tggactctcg caggagcaga 60
tcaagccggg cgaacagttt ctctataaat ggaaggcaag ccagtatggc tcgtacattt 120
atcactcgca cacacgggct cagattgatg atggcctcta tggggctatc tacattgaac 180
cggcagactc cgtggagagg cctttccatt tgatcgctgg atctgatgcg gatgagcagc 240
aggcgatgtt ggcagctgaa aagaatacac ggccagttct catctcggat tggcgtgcct 300
tttcctcgca tgatattctc cagattcaaa ccgaatctgg tgttgaggcc tactgtgcca 360
actctgtttt gatcaacgga aagggctcgg tgatttgccc gtctcaggaa cacatcaatg 420
ctgccacgac accgcagcaa aagcagatcc tgggcaactt gactctgact gatatggggt 480
atgaagtccc aagacttgat gacggttaca atgctgatcc tggctagatg cctgcctccc 540
acgccgggtg ttgtgggtcc gtacccgtac gacctcagca agattcccaa aggtttctac 600
gaaggatgca cacctagtga agggcccaca gaggttttca aggtcaactc ctcatcgcgg 660
tatgctagct ttgactttat tagtatggct ggctcgactt cgcttgtgtt ctccatcgat 720
gagcatccga tgtatgttta cgcggtcgat ggccgttatg ttgaaccttt gcttgtcgaa 780
gctgttactg tgcctgtcgc ttcaagatac tcagttatgg tgccactcaa gtcggaagac 840
caggcaggag attatactat ccgcgttgcc aataactatg cgaaccagct catcaacggg 900
accgccgtcc tgtcgtatga caccgctaca ccgaagcaga tcggtacctc gcagccttat 960
atcaacgagg ctggcgctaa tgccactgcc tccacggtca ttttgaacga aaccgacgtg 1020
atacccttcc ccgtggtagc acctgctaag aaggcggacc ggacttacat cctgaatgtc 1080
gaaaatgcca attcatccta cacctggacc ctgggcaacc agtaccccgt gtcgaatgaa 1140
gagcttcgcc ctcctgtctt gttcaacctc agctcaatct cgcaggcgta ctcggccatg 1200
acggagtacg gtacctgggt tgatctcatc atcaacatca ccacctcggg ccagcctcaa 1260
caccccatcc acaagcactc caacaagtac ttcgtgatcg gaaccggtaa ccagcccttc 1320
atctggtcct ccgtggaaga ggcaatgaaa gacataccgg agaatttcaa cttcgaaaac 1380

ccccagatgc gggacacttt ctactcgcca agctcaagca cagggccgag ctggctggca 1440

atgcgctacc atatcgtgaa cccagggcca ttcctacttc attgccatct tcagatgcac 1500

catgtcggtg ggttggcact ggccttgctg gacggggtgg acgcctggcc aaccgacatt 1560

cccgagggat atcggttgcc agtgatgcca atctag 1596

<210> 12
<211> 612
<212> DNA
<213> Aspergillus ficuum

<400> 12
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggttttttga 360

gccccagcat gagtatatct caaagcaggc tggccctgtt ggccctttgc ttcgtgcaat 420

gggtgtccgg ccgtgttgtg cagttccagc tagacctgac ctatgaagat gtcagtgtcg 480

ctggtgatgt acacaaggct attgtgagta atggacagat tcctgggcct acgttgtggt 540

tgaagcaggg tgatgatgtc gaatttttgg tcaacaactc gatgtcaatc aatactacgg 600

ttcattttca tg 612

<210> 13
<211> 697
<212> DNA
<213> Aspergillus ficuum

<400> 13
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggtttttga 360

gccccagcat gagtatatct caaagcaggc tggccctgtt ggccctttgc ttcgtgcaat 420

gggtgtccgg ccgtgttgtg cagttccagc tagacctgac ctatgaagat gtcagtgtcg 480

ctggtgatgt acacaaggct attgtgagta atggacagat tcctgggcct acgttgtggt 540

tgaagcaggg tgatgatgtc gaatttttgg tcaacaactc gatgtcaatc aatactacgg 600

ttcattttca tggtgagtac tacctctcga ttgtatgatc ctttggcttc gatgttcaga 660

acgccttttc ctatagagct ttactaaaga tttgtag 697

<210> 14
<211> 1175
<212> DNA
<213> Aspergillus ficuum

<400> 14
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggttttttga 360

gccccagcat gagtatatct caaagcaggc tggccctgtt ggccctttgc ttcgtgcaat 420

gggtgtccgg ccgtgttgtg cagttccagc tagacctgac ctatgaagat gtcagtgtcg 480

ctggtgatgt acacaaggct attgtgagta atggacagat tcctgggcct acgttgtggt 540

tgaagcaggg tgatgatgtc gaatttttgg tcaacaactc gatgtcaatc aatactacgg 600

ttcattttca tggtgagtac tacctctcga ttgtatgatc ctttggcttc gatgttcaga 660

acgccttttc ctatagagct ttactaaaga tttgtaggta tcgagcaact aggcaccccg 720

tggtcagatg gtgtacctgg actctcgcag gagcagatca agccgggcga acagtttctc 780

tataaatgga aggcaagcca gtatggctcg tacatttatc actcgcacac acgggctcag 840

attgatgatg gcctctatgg ggctatctac attgaaccgg cagactccgt ggagaggcct 900

ttccatttga tcgctggatc tgatgcggat gagcagcagg cgatgttggc agctgaaaag 960

aatacacggc cagttctcat ctcggattgg cgtgcctttt cctcgcatga tattctccag 1020

attcaaaccg aatctggtgt tgaggcctac tgtgccaact ctgttttgat caacggaaag 1080

ggctcggtga tttgcccgtc tcaggaacac atcaatgctg ccacgacacc gcagcaaaag 1140

cagatcctgg gcaacttgac tctgactgat atggg 1175

<210> 15
<211> 1224
<212> DNA
<213> Aspergillus ficuum

<400> 15
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggtttttga 360

gccccagcat gagtatatct caaagcaggc tggccctgtt ggccctttgc ttcgtgcaat 420

gggtgtccgg ccgtgttgtg cagttccagc tagacctgac ctatgaagat gtcagtgtcg 480

ctggtgatgt acacaaggct attgtgagta atggacagat tcctgggcct acgttgtggt 540

tgaagcaggg tgatgatgtc gaatttttgg tcaacaactc gatgtcaatc aatactacgg 600

ttcattttca tggtgagtac tacctctcga ttgtatgatc ctttggcttc gatgttcaga 660

acgccttttc ctatagagct ttactaaaga tttgtaggta tcgagcaact aggcaccccg 720

tggtcagatg gtgtacctgg actctcgcag gagcagatca agccgggcga acagtttctc 780

tataaatgga aggcaagcca gtatggctcg tacatttatc actcgcacac acgggctcag 840

attgatgatg gcctctatgg ggctatctac attgaaccgg cagactccgt ggagaggcct 900

ttccatttga tcgctggatc tgatgcggat gagcagcagg cgatgttggc agctgaaaag 960

aatacacggc cagttctcat ctcggattgg cgtgcctttt cctcgcatga tattctccag 1020

attcaaaccg aatctggtgt tgaggcctac tgtgccaact ctgttttgat caacggaaag 1080

ggctcggtga tttgcccgtc tcaggaacac atcaatgctg ccacgacacc gcagcaaaag 1140

cagatcctgg gcaacttgac tctgactgat atggggtatg aagtcccaag acttgatgac 1200

ggttacaatg ctgatcctgg ctag 1224

<210> 16
<211> 63
<212> DNA
<213> Aspergillus ficuum

<400> 16
atgagtatat ctcaaagcag gctggccctg ttggcccttt gcttcgtgca atgggtgtcc 60

ggc 63

<210> 17
<211> 244
<212> DNA
<213> Aspergillus ficuum

<400> 17
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catg 244

<210> 18
<211> 329
<212> DNA
<213> Aspergillus ficuum

<400> 18
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtag 329

<210> 19
<211> 807
<212> DNA
<213> Aspergillus ficuum

<400> 19
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtagg tatcgagcaa ctaggcaccc cgtggtcaga 360

tggtgtacct ggactctcgc aggagcagat caagccgggc gaacagtttc tctataaatg 420

gaaggcaagc cagtatggct cgtacattta tcactcgcac acacgggctc agattgatga 480

tggcctctat ggggctatct acattgaacc ggcagactcc gtggagaggc ctttccattt 540

gatcgctgga tctgatgcgg atgagcagca ggcgatgttg gcagctgaaa agaatacacg 600

gccagttctc atctcggatt ggcgtgcctt ttcctcgcat gatattctcc agattcaaac 660

cgaatctggt gttgaggcct actgtgccaa ctctgttttg atcaacggaa agggctcggt 720

gatttgcccg tctcaggaac acatcaatgc tgccacgaca ccgcagcaaa agcagatcct 780

gggcaacttg actctgactg atatggg 807

<210> 20
<211> 856
<212> DNA
<213> Aspergillus ficuum

<400> 20
atgagtatat ctcaaagcag gctggccctg ttggcccttt gcttcgtgca atgggtgtcc 60

ggccgtgttg tgcagttcca gctagacctg acctatgaag atgtcagtgt cgctggtgat 120

gtacacaagg ctattgtgag taatggacag attcctgggc ctacgttgtg gttgaagcag 180

ggtgatgatg tcgaattttt ggtcaacaac tcgatgtcaa tcaatactac ggttcatttt 240

catggtgagt actacctctc gattgtatga tcctttggct tcgatgttca gaacgccttt 300

tcctatagag ctttactaaa gatttgtagg tatcgagcaa ctaggcaccc cgtggtcaga 360

tggtgtacct ggactctcgc aggagcagat caagccgggc gaacagtttc tctataaatg 420

gaaggcaagc cagtatggct cgtacattta tcactcgcac acacgggctc agattgatga 480

tggcctctat ggggctatct acattgaacc ggcagactcc gtggagaggc ctttccattt 540

gatcgctgga tctgatgcgg atgagcagca ggcgatgttg gcagctgaaa agaatacacg 600

gccagttctc atctcggatt ggcgtgcctt ttcctcgcat gatattctcc agattcaaac 660

cgaatctggt gttgaggcct actgtgccaa ctctgttttg atcaacggaa agggctcggt 720

gatttgcccg tctcaggaac acatcaatgc tgccacgaca ccgcagcaaa agcagatcct 780

gggcaacttg actctgactg atatggggta tgaagtccca agacttgatg acggttacaa 840

tgctgatcct ggctag 856

<210> 21
<211> 563
<212> DNA
<213> Aspergillus ficuum

<400> 21
gtgagtacta cctctcgatt gtatgatcct ttggcttcga tgttcagaac gccttttcct 60

atagagcttt actaaagatt tgtaggtatc gagcaactag gcaccccgtg gtcagatggt 120

gtacctggac tctcgcagga gcagatcaag ccgggcgaac agtttctcta taaatggaag 180

gcaagccagt atggctcgta catttatcac tcgcacacac gggctcagat tgatgatggc 240

ctctatgggg ctatctacat tgaaccggca gactccgtgg agaggccttt ccatttgatc 300

gctggatctg atgcggatga gcagcaggcg atgttggcag ctgaaaagaa tacacggcca 360

gttctcatct cggattggcg tgccttttcc tcgcatgata ttctccagat tcaaaccgaa 420

tctggtgttg aggcctactg tgccaactct gttttgatca acggaaaggg ctcggtgatt 480

tgcccgtctc aggaacacat caatgctgcc acgacaccgc agcaaaagca gatcctgggc 540

aacttgactc tgactgatat ggg 563

<210> 22
<211> 612
<212> DNA
<213> Aspergillus ficuum

<400> 22
gtgagtacta cctctcgatt gtatgatcct ttggcttcga tgttcagaac gccttttcct 60

atagagcttt actaaagatt tgtaggtatc gagcaactag gcaccccgtg gtcagatggt 120

gtacctggac tctcgcagga gcagatcaag ccgggcgaac agtttctcta taaatggaag 180

gcaagccagt atggctcgta catttatcac tcgcacacac gggctcagat tgatgatggc 240

ctctatgggg ctatctacat tgaaccggca gactccgtgg agaggccttt ccatttgatc 300

gctggatctg atgcggatga gcagcaggcg atgttggcag ctgaaaagaa tacacggcca 360

gttctcatct cggattggcg tgccttttcc tcgcatgata ttctccagat tcaaaccgaa 420

tctggtgttg aggcctactg tgccaactct gttttgatca acggaaaggg ctcggtgatt 480

tgcccgtctc aggaacacat caatgctgcc acgacaccgc agcaaaagca gatcctgggc 540

aacttgactc tgactgatat ggggtatgaa gtcccaagac ttgatgacgg ttacaatgct 600

gatcctggct ag 612

<210> 23
<211> 1681
<212> DNA
<213> Aspergillus ficuum

<400> 23
gtgagtacta cctctcgatt gtatgatcct ttggcttcga tgttcagaac gccttttcct 60

atagagcttt actaaagatt tgtaggtatc gagcaactag gcaccccgtg gtcagatggt 120

gtacctggac tctcgcagga gcagatcaag ccgggcgaac agtttctcta taaatggaag 180

gcaagccagt atggctcgta catttatcac tcgcacacac gggctcagat tgatgatggc 240

ctctatgggg ctatctacat tgaaccggca gactccgtgg agaggccttt ccatttgatc 300

gctggatctg atgcggatga gcagcaggcg atgttggcag ctgaaaagaa tacacggcca 360

gttctcatct cggattggcg tgccttttcc tcgcatgata ttctccagat tcaaaccgaa 420

tctggtgttg aggcctactg tgccaactct gttttgatca acggaaaggg ctcggtgatt 480

tgcccgtctc aggaacacat caatgctgcc acgacaccgc agcaaaagca gatcctgggc 540

aacttgactc tgactgatat ggggtatgaa gtcccaagac ttgatgacgg ttacaatgct 600

gatcctggct agatgcctgc ctcccacgcc gggtgttgtg ggtccgtacc cgtacgacct 660

cagcaagatt cccaaaggtt tctacgaagg atgcacacct agtgaagggc ccacagaggt 720

tttcaaggtc aactcctcat cgcggtatgc tagctttgac tttattagta tggctggctc 780

gacttcgctt gtgttctcca tcgatgagca tccgatgtat gtttacgcgg tcgatggccg 840

ttatgttgaa cctttgcttg tcgaagctgt tactgtgcct gtcgcttcaa gatactcagt 900

tatggtgcca ctcaagtcgg aagaccaggc aggagattat actatccgcg ttgccaataa 960

ctatgcgaac cagctcatca acgggaccgc cgtcctgtcg tatgacaccg ctacaccgaa 1020

gcagatcggt acctcgcagc cttatatcaa cgaggctggc gctaatgcca ctgcctccac 1080

ggtcattttg aacgaaaccg acgtgatacc cttccccgtg gtagcacctg ctaagaaggc 1140

ggaccggact tacatcctga atgtcgaaaa tgccaattca tcctacacct ggaccctggg 1200

caaccagtac cccgtgtcga atgaagagct tcgccctcct gtcttgttca acctcagctc 1260

aatctcgcag gcgtactcgg ccatgacgga gtacggtacc tgggttgatc tcatcatcaa 1320

catcaccacc tcgggccagc ctcaacaccc catccacaag cactccaaca agtacttcgt 1380

gatcggaacc ggtaaccagc ccttcatctg gtcctccgtg gaagaggcaa tgaaagacat 1440

accggagaat ttcaacttcg aaaaccccca gatgcgggac actttctact cgccaagctc 1500

aagcacaggg ccgagctggc tggcaatgcg ctaccatatc gtgaacccag ggccattcct 1560

acttcattgc catcttcaga tgcaccatgt cggtgggttg gcactggcct tgctggacgg 1620

ggtggacgcc tggccaaccg acattcccga gggatatcgg ttgccagtga tgccaatcta 1680

g 1681

<210> 24
<211> 1875
<212> DNA
<213> Aspergillus ficuum

<400> 24
gtgagtacta cctctcgatt gtatgatcct ttggcttcga tgttcagaac gccttttcct 60

atagagcttt actaaagatt tgtaggtatc gagcaactag gcaccccgtg gtcagatggt 120
gtacctggac tctcgcagga gcagatcaag ccgggcgaac agtttctcta taaatggaag 180
gcaagccagt atggctcgta catttatcac tcgcacacac gggctcagat tgatgatggc 240
ctctatgggg ctatctacat tgaaccggca gactccgtgg agaggccttt ccatttgatc 300
gctggatctg atgcggatga gcagcaggcg atgttggcag ctgaaaagaa tacacggcca 360
gttctcatct cggattggcg tgccttttcc tcgcatgata ttctccagat tcaaaccgaa 420
tctggtgttg aggcctactg tgccaactct gttttgatca acggaaaggg ctcggtgatt 480
tgcccgtctc aggaacacat caatgctgcc acgacaccgc agcaaaagca gatcctgggc 540
aacttgactc tgactgatat ggggtatgaa gtcccaagac ttgatgacgg ttacaatgct 600
gatcctggct agatgcctgc ctcccacgcc gggtgttgtg ggtccgtacc cgtacgacct 660
cagcaagatt cccaaaggtt tctacgaagg atgcacacct agtgaagggc ccacagaggt 720
tttcaaggtc aactcctcat cgcggtatgc tagctttgac tttattagta tggctggctc 780
gacttcgctt gtgttctcca tcgatgagca tccgatgtat gtttacgcgg tcgatggccg 840
ttatgttgaa cctttgcttg tcgaagctgt tactgtgcct gtcgcttcaa gatactcagt 900
tatggtgcca ctcaagtcgg aagaccaggc aggagattat actatccgcg ttgccaataa 960
ctatgcgaac cagctcatca acgggaccgc cgtcctgtcg tatgacaccg ctacaccgaa 1020
gcagatcggt acctcgcagc cttatatcaa cgaggctggc gctaatgcca ctgcctccac 1080
ggtcattttg aacgaaaccg acgtgatacc cttccccgtg gtagcacctg ctaagaaggc 1140
ggaccggact tacatcctga atgtcgaaaa tgccaattca tcctacacct ggaccctggg 1200
caaccagtac cccgtgtcga atgaagagct tcgccctcct gtcttgttca acctcagctc 1260
aatctcgcag gcgtactcgg ccatgacgga gtacggtacc tgggttgatc tcatcatcaa 1320
catcaccacc tcgggccagc ctcaacaccc catccacaag cactccaaca agtacttcgt 1380
gatcggaacc ggtaaccagc ccttcatctg gtcctccgtg gaagaggcaa tgaaagacat 1440
accggagaat ttcaacttcg aaaaccccca gatgcgggac actttctact cgccaagctc 1500
aagcacaggg ccgagctggc tggcaatgcg ctaccatatc gtgaacccag ggccattcct 1560
acttcattgc catcttcaga tgcaccatgt cggtgggttg gcactggcct tgctggacgg 1620
ggtggacgcc tggccaaccg acattcccga gggatatcgg ttgccagtga tgccaatcta 1680
gggactttga ctcaattgta cagaggtgga cgagagtatc tacttgtaat atggccatat 1740
agcgcctctc atatagatcc ggtgtaatat tatttattta ctacataatg aacatgcttg 1800
gtgacttacc aattccccag ttatggctcc aaagtaaaag tgagagcaat cacaccaaat 1860
caggcacagc agtaa 1875

<210> 25
<211> 478
<212> DNA
<213> Aspergillus ficuum

<400> 25

gtatcgagca actaggcacc ccgtggtcag atggtgtacc tggactctcg caggagcaga 60

tcaagccggg cgaacagttt ctctataaat ggaaggcaag ccagtatggc tcgtacattt 120

atcactcgca cacacgggct cagattgatg atggcctcta tggggctatc tacattgaac 180

cggcagactc cgtggagagg cctttccatt tgatcgctgg atctgatgcg gatgagcagc 240

aggcgatgtt ggcagctgaa aagaatacac ggccagttct catctcggat tggcgtgcct 300

tttcctcgca tgatattctc cagattcaaa ccgaatctgg tgttgaggcc tactgtgcca 360

actctgtttt gatcaacgga aagggctcgg tgatttgccc gtctcaggaa cacatcaatg 420

ctgccacgac accgcagcaa aagcagatcc tgggcaactt gactctgact gatatggg 478

<210> 26
<211> 527
<212> DNA
<213> Aspergillus ficuum

<400> 26
gtatcgagca actaggcacc ccgtggtcag atggtgtacc tggactctcg caggagcaga 60

tcaagccggg cgaacagttt ctctataaat ggaaggcaag ccagtatggc tcgtacattt 120

atcactcgca cacacgggct cagattgatg atggcctcta tggggctatc tacattgaac 180

cggcagactc cgtggagagg cctttccatt tgatcgctgg atctgatgcg gatgagcagc 240

aggcgatgtt ggcagctgaa aagaatacac ggccagttct catctcggat tggcgtgcct 300

tttcctcgca tgatattctc cagattcaaa ccgaatctgg tgttgaggcc tactgtgcca 360

actctgtttt gatcaacgga aagggctcgg tgatttgccc gtctcaggaa cacatcaatg 420

ctgccacgac accgcagcaa aagcagatcc tgggcaactt gactctgact gatatggggt 480

atgaagtccc aagacttgat gacggttaca atgctgatcc tggctag 527

<210> 27
<211> 1596
<212> DNA
<213> Aspergillus ficuum

<400> 27
gtatcgagca actaggcacc ccgtggtcag atggtgtacc tggactctcg caggagcaga 60

tcaagccggg cgaacagttt ctctataaat ggaaggcaag ccagtatggc tcgtacattt 120

atcactcgca cacacgggct cagattgatg atggcctcta tggggctatc tacattgaac 180

cggcagactc cgtggagagg cctttccatt tgatcgctgg atctgatgcg gatgagcagc 240

aggcgatgtt ggcagctgaa aagaatacac ggccagttct catctcggat tggcgtgcct 300

tttcctcgca tgatattctc cagattcaaa ccgaatctgg tgttgaggcc tactgtgcca 360

actctgtttt gatcaacgga aagggctcgg tgatttgccc gtctcaggaa cacatcaatg 420

ctgccacgac accgcagcaa aagcagatcc tgggcaactt gactctgact gatatggggt 480

atgaagtccc aagacttgat gacggttaca atgctgatcc tggctagatg cctgcctccc 540

acgccgggtg ttgtgggtcc gtacccgtac gacctcagca agattcccaa aggtttctac 600

gaaggatgca cacctagtga agggcccaca gaggttttca aggtcaactc ctcatcgcgg 660

tatgctagct ttgactttat tagtatggct ggctcgactt cgcttgtgtt ctccatcgat 720
gagcatccga tgtatgttta cgcggtcgat ggccgttatg ttgaaccttt gcttgtcgaa 780
gctgttactg tgcctgtcgc ttcaagatac tcagttatgg tgccactcaa gtcggaagac 840
caggcaggag attatactat ccgcgttgcc aataactatg cgaaccagct catcaacggg 900
accgccgtcc tgtcgtatga caccgctaca ccgaagcaga tcggtacctc gcagccttat 960
atcaacgagg ctggcgctaa tgccactgcc tccacggtca ttttgaacga aaccgacgtg 1020
atacccttcc ccgtggtagc acctgctaag aaggcggacc ggacttacat cctgaatgtc 1080
gaaaatgcca attcatccta cacctggacc ctgggcaacc agtaccccgt gtcgaatgaa 1140
gagcttcgcc ctcctgtctt gttcaacctc agctcaatct cgcaggcgta ctcggccatg 1200
acggagtacg gtacctgggt tgatctcatc atcaacatca ccacctcggg ccagcctcaa 1260
caccccatcc acaagcactc caacaagtac ttcgtgatcg gaaccggtaa ccagcccttc 1320
atctggtcct ccgtggaaga ggcaatgaaa gacataccgg agaatttcaa cttcgaaaac 1380
ccccagatgc gggacacttt ctactcgcca agctcaagca cagggccgag ctggctggca 1440
atgcgctacc atatcgtgaa cccagggcca ttcctacttc attgccatct tcagatgcac 1500
catgtcggtg ggttggcact ggccttgctg gacggggtgg acgcctggcc aaccgacatt 1560
cccgagggat atcggttgcc agtgatgcca atctag 1596

<210> 28
<211> 1790
<212> DNA
<213> Aspergillus ficuum

<400> 28
gtatcgagca actaggcacc ccgtggtcag atggtgtacc tggactctcg caggagcaga 60
tcaagccggg cgaacagttt ctctataaat ggaaggcaag ccagtatggc tcgtacattt 120
atcactcgca cacacgggct cagattgatg atggcctcta tggggctatc tacattgaac 180
cggcagactc cgtggagagg cctttccatt tgatcgctgg atctgatgcg gatgagcagc 240
aggcgatgtt ggcagctgaa aagaatacac ggccagttct catctcggat tggcgtgcct 300
tttcctcgca tgatattctc cagattcaaa ccgaatctgg tgttgaggcc tactgtgcca 360
actctgtttt gatcaacgga aagggctcgg tgatttgccc gtctcaggaa cacatcaatg 420
ctgccacgac accgcagcaa aagcagatcc tgggcaactt gactctgact gatatggggt 480
atgaagtccc aagacttgat gacggttaca atgctgatcc tggctagatg cctgcctccc 540
acgccgggtg ttgtgggtcc gtacccgtac gacctcagca agattcccaa aggtttctac 600
gaaggatgca cacctagtga agggcccaca gaggttttca aggtcaactc ctcatcgcgg 660
tatgctagct ttgactttat tagtatggct ggctcgactt cgcttgtgtt ctccatcgat 720
gagcatccga tgtatgttta cgcggtcgat ggccgttatg ttgaaccttt gcttgtcgaa 780
gctgttactg tgcctgtcgc ttcaagatac tcagttatgg tgccactcaa gtcggaagac 840
caggcaggag attatactat ccgcgttgcc aataactatg cgaaccagct catcaacggg 900

accgccgtcc tgtcgtatga caccgctaca ccgaagcaga tcggtacctc gcagccttat 960
atcaacgagg ctggcgctaa tgccactgcc tccacggtca ttttgaacga aaccgacgtg 1020
atacccttcc ccgtggtagc acctgctaag aaggcggacc ggacttacat cctgaatgtc 1080
gaaaatgcca attcatccta cacctggacc ctgggcaacc agtaccccgt gtcgaatgaa 1140
gagcttcgcc ctcctgtctt gttcaacctc agctcaatct cgcaggcgta ctcggccatg 1200
acggagtacg gtacctgggt tgatctcatc atcaacatca ccacctcggg ccagcctcaa 1260
caccccatcc acaagcactc caacaagtac ttcgtgatcg gaaccggtaa ccagcccttc 1320
atctggtcct ccgtggaaga ggcaatgaaa gacataccgg agaatttcaa cttcgaaaac 1380
ccccagatgc gggacacttt ctactcgcca agctcaagca cagggccgag ctggctggca 1440
atgcgctacc atatcgtgaa cccagggcca ttcctacttc attgccatct tcagatgcac 1500
catgtcggtg ggttggcact ggccttgctg gacggggtgg acgcctggcc aaccgacatt 1560
cccgagggat atcggttgcc agtgatgcca atctagggac tttgactcaa ttgtacagag 1620
gtggacgaga gtatctactt gtaatatggc catatagcgc ctctcatata gatccggtgt 1680
aatattattt atttactaca taatgaacat gcttggtgac ttaccaattc cccagttatg 1740
gctccaaagt aaaagtgaga gcaatcacac caaatcaggc acagcagtaa 1790

<210> 29
<211> 1118
<212> DNA
<213> Aspergillus ficuum

<400> 29
gtatgaagtc ccaagacttg atgacggtta caatgctgat cctggctaga tgcctgcctc 60
ccacgccggg tgttgtgggt ccgtacccgt acgacctcag caagattccc aaaggtttct 120
acgaaggatg cacacctagt gaagggccca cagaggtttt caaggtcaac tcctcatcgc 180
ggtatgctag ctttgacttt attagtatgg ctggctcgac ttcgcttgtg ttctccatcg 240
atgagcatcc gatgtatgtt tacgcggtcg atggccgtta tgttgaacct ttgcttgtcg 300
aagctgttac tgtgcctgtc gcttcaagat actcagttat ggtgccactc aagtcggaag 360
accaggcagg agattatact atccgcgttg ccaataacta tgcgaaccag ctcatcaacg 420
ggaccgccgt cctgtcgtat gacaccgcta caccgaagca gatcggtacc tcgcagcctt 480
atatcaacga ggctggcgct aatgccactg cctccacggt cattttgaac gaaaccgacg 540
tgataccctt ccccgtggta gcacctgcta agaaggcgga ccggacttac atcctgaatg 600
tcgaaaatgc caattcatcc tacacctgga ccctgggcaa ccagtacccc gtgtcgaatg 660
aagagcttcg ccctcctgtc ttgttcaacc tcagctcaat ctcgcaggcg tactcggcca 720
tgacggagta cggtacctgg gttgatctca tcatcaacat caccacctcg ggccagcctc 780
aacaccccat ccacaagcac tccaacaagt acttcgtgat cggaaccggt aaccagccct 840
tcatctggtc ctccgtggaa gaggcaatga aagacatacc ggagaatttc aacttcgaaa 900
acccccagat gcgggacact ttctactcgc caagctcaag cacagggccg agctggctgg 960

caatgcgcta ccatatcgtg aacccagggc cattcctact tcattgccat cttcagatgc 1020

accatgtcgg tgggttggca ctggccttgc tggacggggt ggacgcctgg ccaaccgaca 1080

ttcccgaggg atatcggttg ccagtgatgc caatctag 1118

<210> 30
<211> 1312
<212> DNA
<213> Aspergillus ficuum

<400> 30
gtatgaagtc ccaagacttg atgacggtta caatgctgat cctggctaga tgcctgcctc 60

ccacgccggg tgttgtgggt ccgtacccgt acgacctcag caagattccc aaaggtttct 120

acgaaggatg cacacctagt gaagggccca cagaggtttt caaggtcaac tcctcatcgc 180

ggtatgctag ctttgacttt attagtatgg ctggctcgac ttcgcttgtg ttctccatcg 240

atgagcatcc gatgtatgtt tacgcggtcg atggccgtta tgttgaacct ttgcttgtcg 300

aagctgttac tgtgcctgtc gcttcaagat actcagttat ggtgccactc aagtcggaag 360

accaggcagg agattatact atccgcgttg ccaataacta tgcgaaccag ctcatcaacg 420

ggaccgccgt cctgtcgtat gacaccgcta caccgaagca gatcggtacc tcgcagcctt 480

atatcaacga ggctggcgct aatgccactg cctccacggt cattttgaac gaaaccgacg 540

tgataccctt ccccgtggta gcacctgcta agaaggcgga ccggacttac atcctgaatg 600

tcgaaaatgc caattcatcc tacacctgga ccctgggcaa ccagtacccc gtgtcgaatg 660

aagagcttcg ccctcctgtc ttgttcaacc tcagctcaat ctcgcaggcg tactcggcca 720

tgacggagta cggtacctgg gttgatctca tcatcaacat caccacctcg ggccagcctc 780

aacaccccat ccacaagcac tccaacaagt acttcgtgat cggaaccggt aaccagccct 840

tcatctggtc ctccgtggaa gaggcaatga aagacatacc ggagaatttc aacttcgaaa 900

acccccagat gcgggacact ttctactcgc caagctcaag cacagggccg agctggctgg 960

caatgcgcta ccatatcgtg aacccagggc cattcctact tcattgccat cttcagatgc 1020

accatgtcgg tgggttggca ctggccttgc tggacggggt ggacgcctgg ccaaccgaca 1080

ttcccgaggg atatcggttg ccagtgatgc caatctaggg actttgactc aattgtacag 1140

aggtggacga gagtatctac ttgtaatatg gccatatagc gcctctcata tagatccggt 1200

gtaatattat ttatttacta cataatgaac atgcttggtg acttaccaat tccccagtta 1260

tggctccaaa gtaaaagtga gagcaatcac accaaatcag gcacagcagt aa 1312

<210> 31
<211> 1069
<212> DNA
<213> Aspergillus ficuum

<400> 31
atgcctgcct cccacgccgg gtgttgtggg tccgtacccg tacgacctca gcaagattcc 60

caaaggtttc tacgaaggat gcacacctag tgaagggccc acagaggttt tcaaggtcaa 120

ctcctcatcg cggtatgcta gctttgactt tattagtatg gctggctcga cttcgcttgt 180
gttctccatc gatgagcatc cgatgtatgt ttacgcggtc gatggccgtt atgttgaacc 240
tttgcttgtc gaagctgtta ctgtgcctgt cgcttcaaga tactcagtta tggtgccact 300
caagtcggaa gaccaggcag gagattatac tatccgcgtt gccaataact atgcgaacca 360
gctcatcaac gggaccgccg tcctgtcgta tgacaccgct acaccgaagc agatcggtac 420
ctcgcagcct tatatcaacg aggctggcgc taatgccact gcctccacgg tcattttgaa 480
cgaaaccgac gtgataccct tccccgtggt agcacctgct aagaaggcgg accggactta 540
catcctgaat gtcgaaaatg ccaattcatc ctacacctgg accctgggca accagtaccc 600
cgtgtcgaat gaagagcttc gccctcctgt cttgttcaac ctcagctcaa tctcgcaggc 660
gtactcggcc atgacggagt acggtacctg ggttgatctc atcatcaaca tcaccacctc 720
gggccagcct caacacccca tccacaagca ctccaacaag tacttcgtga tcggaaccgg 780
taaccagccc ttcatctggt cctccgtgga agaggcaatg aaagacatac cggagaattt 840
caacttcgaa aacccccaga tgcgggacac tttctactcg ccaagctcaa gcacagggcc 900
gagctggctg gcaatgcgct accatatcgt gaacccaggg ccattcctac ttcattgcca 960
tcttcagatg caccatgtcg gtgggttggc actggccttg ctggacgggg tggacgcctg 1020
gccaaccgac attcccgagg gatatcggtt gccagtgatg ccaatctag 1069

<210> 32
<211> 1263
<212> DNA
<213> Aspergillus ficuum

<400> 32
atgcctgcct cccacgccgg gtgttgtggg tccgtacccg tacgacctca gcaagattcc 60
caaaggtttc tacgaaggat gcacacctag tgaagggccc acagaggttt tcaaggtcaa 120
ctcctcatcg cggtatgcta gctttgactt tattagtatg gctggctcga cttcgcttgt 180
gttctccatc gatgagcatc cgatgtatgt ttacgcggtc gatggccgtt atgttgaacc 240
tttgcttgtc gaagctgtta ctgtgcctgt cgcttcaaga tactcagtta tggtgccact 300
caagtcggaa gaccaggcag gagattatac tatccgcgtt gccaataact atgcgaacca 360
gctcatcaac gggaccgccg tcctgtcgta tgacaccgct acaccgaagc agatcggtac 420
ctcgcagcct tatatcaacg aggctggcgc taatgccact gcctccacgg tcattttgaa 480
cgaaaccgac gtgataccct tccccgtggt agcacctgct aagaaggcgg accggactta 540
catcctgaat gtcgaaaatg ccaattcatc ctacacctgg accctgggca accagtaccc 600
cgtgtcgaat gaagagcttc gccctcctgt cttgttcaac ctcagctcaa tctcgcaggc 660
gtactcggcc atgacggagt acggtacctg ggttgatctc atcatcaaca tcaccacctc 720
gggccagcct caacacccca tccacaagca ctccaacaag tacttcgtga tcggaaccgg 780
taaccagccc ttcatctggt cctccgtgga agaggcaatg aaagacatac cggagaattt 840
caacttcgaa aacccccaga tgcgggacac tttctactcg ccaagctcaa gcacagggcc 900

gagctggctg gcaatgcgct accatatcgt gaacccaggg ccattcctac ttcattgcca 960

tcttcagatg caccatgtcg gtgggttggc actggccttg ctggacgggg tggacgcctg 1020

gccaaccgac attcccgagg gatatcggtt gccagtgatg ccaatctagg gactttgact 1080

caattgtaca gaggtggacg agagtatcta cttgtaatat ggccatatag cgcctctcat 1140

atagatccgg tgtaatatta tttatttact acataatgaa catgcttggt gacttaccaa 1200

ttccccagtt atggctccaa agtaaaagtg agagcaatca caccaaatca ggcacagcag 1260

taa 1263

<210> 33
<211> 368
<212> DNA
<213> Aspergillus ficuum

<400> 33
taattgaagc ttgcggtgag caagacagtc tgcaccctgc tagaaatgtt cgtatgccgg 60

taggccctgc atatgacgga tccagtatct tggccatcgg acggcattat gacaagccct 120

agtcatgaac ctttcgctat acgtctcggg tcctccatcc cttgccttga ccttcgggca 180

tcaatggcat caggtcaatg tgcctcttga gttctgtcat tataaaagcc tcaaacttct 240

cgcaggatga agggaactca accacgacaa accagtccgc gactcggccc tgttcgactg 300

tatatattct gtggaactag gtagttggtt ccttgtatat ttctttattt ggttttttga 360

gccccagc 368

<210> 34
<211> 85
<212> DNA
<213> Aspergillus ficuum

<400> 34
gtgagtacta cctctcgatt gtatgatcct ttggcttcga tgttcagaac gccttttcct 60

atagagcttt actaaagatt tgtag 85

<210> 35
<211> 49
<212> DNA
<213> Aspergillus ficuum

<400> 35
gtatgaagtc ccaagacttg atgacggtta caatgctgat cctggctag 49

<210> 36
<211> 194
<212> DNA
<213> Aspergillus ficuum

<400> 36
ggactttgac tcaattgtac agaggtggac gagagtatct acttgtaata tggccatata 60

gcgcctctca tatagatccg gtgtaatatt atttatttac tacataatga acatgcttgg 120

tgacttacca attccccagt tatggctcca aagtaaaagt gagagcaatc acaccaaatc 180

aggcacagca gtaa 194

<210>  37
<211>  26
<212>  DNA
<213>  Aspergillus ficuum


<220>
<221>  modified_base
<222>  (3)..(3)
<223>  I

<220>
<221>  misc_feature
<222>  (3)..(3)
<223>  n is a, c, g, or t

<220>
<221>  modified_base
<222>  (6)..(6)
<223>  I

<220>
<221>  misc_feature
<222>  (6)..(6)
<223>  n is a, c, g, or t

<220>
<221>  modified_base
<222>  (18)..(18)
<223>  I

<220>
<221>  misc_feature
<222>  (18)..(18)
<223>  n is a, c, g, or t

<220>
<221>  modified_base
<222>  (24)..(24)
<223>  I

<220>
<221>  misc_feature
<222>  (24)..(24)
<223>  n is a, c, g, or t

<400>  37
gtngtncagt ttcagytnga tytnac                                          26


<210>  38
<211>  29
<212>  DNA
<213>  Aspergillus ficuum


<220>
<221>  modified_base
<222>  (12)..(12)
<223>  I

<220>
<221>  misc_feature
<222>  (12)..(12)
<223>  n is a, c, g, or t

<220>

<221>  modified_base
<222>  (15)..(15)
<223>  I

<220>
<221>  misc_feature
<222>  (15)..(15)
<223>  n is a, c, g, or t

<220>
<221>  modified_base
<222>  (18)..(18)
<223>  I

<220>
<221>  misc_feature
<222>  (18)..(18)
<223>  n is a, c, g, or t

<220>
<221>  modified_base
<222>  (27)..(27)
<223>  I

<220>
<221>  misc_feature
<222>  (27)..(27)
<223>  n is a, c, g, or t

<400>  38
ctyctrgtyc gnccnccnct gatgtgnta                                          29


<210>  39
<211>  20
<212>  DNA
<213>  Aspergillus ficuum

<400>  39
caacccaggt accgtactcc                                                    20


<210>  40
<211>  596
<212>  PRT
<213>  Aspergillus ficuum

<400>  40

Met Ser Ile Ser Gln Ser Arg Leu Ala Leu Leu Ala Leu Cys Phe Val
1               5                   10                  15


Gln Trp Val Ser Gly Arg Val Val Gln Phe Gln Leu Asp Leu Thr Tyr
            20                  25                  30


Glu Asp Val Ser Val Ala Gly Asp Val His Lys Ala Ile Val Ser Asn
        35                  40                  45


Gly Gln Ile Pro Gly Pro Thr Leu Trp Leu Lys Gln Gly Asp Asp Val
    50                  55                  60


Glu Phe Leu Val Asn Asn Ser Met Ser Ile Asn Thr Thr Val His Phe
65                  70                  75                  80

His Gly Ile Glu Gln Leu Gly Thr Pro Trp Ser Asp Gly Val Pro Gly
                85                  90                  95

Leu Ser Gln Glu Gln Ile Lys Pro Gly Glu Gln Phe Leu Tyr Lys Trp
            100                 105                 110

Lys Ala Ser Gln Tyr Gly Ser Tyr Ile Tyr His Ser His Thr Arg Ala
        115                 120                 125

Gln Ile Asp Asp Gly Leu Tyr Gly Ala Ile Tyr Ile Glu Pro Ala Asp
    130                 135                 140

Ser Val Glu Arg Pro Phe His Leu Ile Ala Gly Ser Asp Ala Asp Glu
145                 150                 155                 160

Gln Gln Ala Met Leu Ala Ala Glu Lys Asn Thr Arg Pro Val Leu Ile
                165                 170                 175

Ser Asp Trp Arg Ala Phe Ser Ser His Asp Ile Leu Gln Ile Gln Thr
            180                 185                 190

Glu Ser Gly Val Glu Ala Tyr Cys Ala Asn Ser Val Leu Ile Asn Gly
        195                 200                 205

Lys Gly Ser Val Ile Cys Pro Ser Gln Glu His Ile Asn Ala Ala Thr
    210                 215                 220

Thr Pro Gln Gln Lys Gln Ile Leu Gly Asn Leu Thr Leu Thr Asp Met
225                 230                 235                 240

Gly Cys Leu Pro Pro Thr Pro Gly Val Val Gly Pro Tyr Pro Tyr Asp
                245                 250                 255

Leu Ser Lys Ile Pro Lys Gly Phe Tyr Glu Gly Cys Thr Pro Ser Glu
            260                 265                 270

Gly Pro Thr Glu Val Phe Lys Val Asn Ser Ser Ser Arg Tyr Ala Ser
        275                 280                 285

Phe Asp Phe Ile Ser Met Ala Gly Ser Thr Ser Leu Val Phe Ser Ile
    290                 295                 300

Asp Glu His Pro Met Tyr Val Tyr Ala Val Asp Gly Arg Tyr Val Glu
305                 310                 315                 320

Pro Leu Leu Val Glu Ala Val Thr Val Pro Val Ala Ser Arg Tyr Ser
                325                 330                 335

Val Met Val Pro Leu Lys Ser Glu Asp Gln Ala Gly Asp Tyr Thr Ile
            340                 345                 350

Arg Val Ala Asn Asn Tyr Ala Asn Gln Leu Ile Asn Gly Thr Ala Val

        355                 360                 365

Leu Ser Tyr Asp Thr Ala Thr Pro Lys Gln Ile Gly Thr Ser Gln Pro
    370                 375                 380

Tyr Ile Asn Glu Ala Gly Ala Asn Ala Thr Ala Ser Thr Val Ile Leu
385                 390                 395                 400

Asn Glu Thr Asp Val Ile Pro Phe Pro Val Val Ala Pro Ala Lys Lys
                405                 410                 415

Ala Asp Arg Thr Tyr Ile Leu Asn Val Glu Asn Ala Asn Ser Ser Tyr
            420                 425                 430

Thr Trp Thr Leu Gly Asn Gln Tyr Pro Val Ser Asn Glu Glu Leu Arg
        435                 440                 445

Pro Pro Val Leu Phe Asn Leu Ser Ser Ile Ser Gln Ala Tyr Ser Ala
    450                 455                 460

Met Thr Glu Tyr Gly Thr Trp Val Asp Leu Ile Ile Asn Ile Thr Thr
465                 470                 475                 480

Ser Gly Gln Pro Gln His Pro Ile His Lys His Ser Asn Lys Tyr Phe
                485                 490                 495

Val Ile Gly Thr Gly Asn Gln Pro Phe Ile Trp Ser Ser Val Glu Glu
            500                 505                 510

Ala Met Lys Asp Ile Pro Glu Asn Phe Asn Phe Glu Asn Pro Gln Met
        515                 520                 525

Arg Asp Thr Phe Tyr Ser Pro Ser Ser Ser Thr Gly Pro Ser Trp Leu
    530                 535                 540

Ala Met Arg Tyr His Ile Val Asn Pro Gly Pro Phe Leu Leu His Cys
545                 550                 555                 560

His Leu Gln Met His His Val Gly Gly Leu Ala Leu Ala Leu Leu Asp
                565                 570                 575

Gly Val Asp Ala Trp Pro Thr Asp Ile Pro Glu Gly Tyr Arg Leu Pro
            580                 585                 590

Val Met Pro Ile
        595

<210> 41
<211> 575
<212> PRT
<213> Aspergillus ficuum

<400> 41

Arg Val Val Gln Phe Gln Leu Asp Leu Thr Tyr Glu Asp Val Ser Val
1               5                   10                  15

Ala Gly Asp Val His Lys Ala Ile Val Ser Asn Gly Gln Ile Pro Gly
            20                  25                  30

Pro Thr Leu Trp Leu Lys Gln Gly Asp Asp Val Glu Phe Leu Val Asn
        35                  40                  45

Asn Ser Met Ser Ile Asn Thr Thr Val His Phe His Gly Ile Glu Gln
    50                  55                  60

Leu Gly Thr Pro Trp Ser Asp Gly Val Pro Gly Leu Ser Gln Glu Gln
65                  70                  75                  80

Ile Lys Pro Gly Glu Gln Phe Leu Tyr Lys Trp Lys Ala Ser Gln Tyr
                85                  90                  95

Gly Ser Tyr Ile Tyr His Ser His Thr Arg Ala Gln Ile Asp Asp Gly
            100                 105                 110

Leu Tyr Gly Ala Ile Tyr Ile Glu Pro Ala Asp Ser Val Glu Arg Pro
        115                 120                 125

Phe His Leu Ile Ala Gly Ser Asp Ala Asp Glu Gln Gln Ala Met Leu
    130                 135                 140

Ala Ala Glu Lys Asn Thr Arg Pro Val Leu Ile Ser Asp Trp Arg Ala
145                 150                 155                 160

Phe Ser Ser His Asp Ile Leu Gln Ile Gln Thr Glu Ser Gly Val Glu
                165                 170                 175

Ala Tyr Cys Ala Asn Ser Val Leu Ile Asn Gly Lys Gly Ser Val Ile
            180                 185                 190

Cys Pro Ser Gln Glu His Ile Asn Ala Ala Thr Thr Pro Gln Gln Lys
        195                 200                 205

Gln Ile Leu Gly Asn Leu Thr Leu Thr Asp Met Gly Cys Leu Pro Pro
    210                 215                 220

Thr Pro Gly Val Val Gly Pro Tyr Pro Tyr Asp Leu Ser Lys Ile Pro
225                 230                 235                 240

Lys Gly Phe Tyr Glu Gly Cys Thr Pro Ser Glu Gly Pro Thr Glu Val
                245                 250                 255

Phe Lys Val Asn Ser Ser Ser Arg Tyr Ala Ser Phe Asp Phe Ile Ser
            260                 265                 270

Met Ala Gly Ser Thr Ser Leu Val Phe Ser Ile Asp Glu His Pro Met
        275                 280                 285

Tyr Val Tyr Ala Val Asp Gly Arg Tyr Val Glu Pro Leu Leu Val Glu
    290                 295                 300

Ala Val Thr Val Pro Val Ala Ser Arg Tyr Ser Val Met Val Pro Leu
305                 310                 315                 320

Lys Ser Glu Asp Gln Ala Gly Asp Tyr Thr Ile Arg Val Ala Asn Asn
                325                 330                 335

Tyr Ala Asn Gln Leu Ile Asn Gly Thr Ala Val Leu Ser Tyr Asp Thr
            340                 345                 350

Ala Thr Pro Lys Gln Ile Gly Thr Ser Gln Pro Tyr Ile Asn Glu Ala
        355                 360                 365

Gly Ala Asn Ala Thr Ala Ser Thr Val Ile Leu Asn Glu Thr Asp Val
    370                 375                 380

Ile Pro Phe Pro Val Val Ala Pro Ala Lys Lys Ala Asp Arg Thr Tyr
385                 390                 395                 400

Ile Leu Asn Val Glu Asn Ala Asn Ser Ser Tyr Thr Trp Thr Leu Gly
                405                 410                 415

Asn Gln Tyr Pro Val Ser Asn Glu Glu Leu Arg Pro Pro Val Leu Phe
            420                 425                 430

Asn Leu Ser Ser Ile Ser Gln Ala Tyr Ser Ala Met Thr Glu Tyr Gly
        435                 440                 445

Thr Trp Val Asp Leu Ile Ile Asn Ile Thr Thr Ser Gly Gln Pro Gln
    450                 455                 460

His Pro Ile His Lys His Ser Asn Lys Tyr Phe Val Ile Gly Thr Gly
465                 470                 475                 480

Asn Gln Pro Phe Ile Trp Ser Ser Val Glu Glu Ala Met Lys Asp Ile
                485                 490                 495

Pro Glu Asn Phe Asn Phe Glu Asn Pro Gln Met Arg Asp Thr Phe Tyr
            500                 505                 510

Ser Pro Ser Ser Ser Thr Gly Pro Ser Trp Leu Ala Met Arg Tyr His
        515                 520                 525

Ile Val Asn Pro Gly Pro Phe Leu Leu His Cys His Leu Gln Met His
    530                 535                 540

His Val Gly Gly Leu Ala Leu Ala Leu Leu Asp Gly Val Asp Ala Trp

545                 550                 555                 560

Pro Thr Asp Ile Pro Glu Gly Tyr Arg Leu Pro Val Met Pro Ile
                565                 570                 575

<210>  42
<211>  21
<212>  PRT
<213>  Aspergillus ficuum

<400>  42

Met Ser Ile Ser Gln Ser Arg Leu Ala Leu Leu Ala Leu Cys Phe Val
1               5                   10                  15

Gln Trp Val Ser Gly
            20

<210>  43
<211>  81
<212>  PRT
<213>  Aspergillus ficuum

<400>  43

Met Ser Ile Ser Gln Ser Arg Leu Ala Leu Leu Ala Leu Cys Phe Val
1               5                   10                  15

Gln Trp Val Ser Gly Arg Val Val Gln Phe Gln Leu Asp Leu Thr Tyr
            20                  25                  30

Glu Asp Val Ser Val Ala Gly Asp Val His Lys Ala Ile Val Ser Asn
        35                  40                  45

Gly Gln Ile Pro Gly Pro Thr Leu Trp Leu Lys Gln Gly Asp Asp Val
    50                  55                  60

Glu Phe Leu Val Asn Asn Ser Met Ser Ile Asn Thr Thr Val His Phe
65                  70                  75                  80

His

<210>  44
<211>  160
<212>  PRT
<213>  Aspergillus ficuum

<400>  44

Gly Ile Glu Gln Leu Gly Thr Pro Trp Ser Asp Gly Val Pro Gly Leu
1               5                   10                  15

Ser Gln Glu Gln Ile Lys Pro Gly Glu Gln Phe Leu Tyr Lys Trp Lys
            20                  25                  30

Ala Ser Gln Tyr Gly Ser Tyr Ile Tyr His Ser His Thr Arg Ala Gln

35                  40                  45

Ile Asp Asp Gly Leu Tyr Gly Ala Ile Tyr Ile Glu Pro Ala Asp Ser
    50                  55                  60

Val Glu Arg Pro Phe His Leu Ile Ala Gly Ser Asp Ala Asp Glu Gln
65                  70                  75                  80

Gln Ala Met Leu Ala Ala Glu Lys Asn Thr Arg Pro Val Leu Ile Ser
                85                  90                  95

Asp Trp Arg Ala Phe Ser Ser His Asp Ile Leu Gln Ile Gln Thr Glu
            100                 105                 110

Ser Gly Val Glu Ala Tyr Cys Ala Asn Ser Val Leu Ile Asn Gly Lys
        115                 120                 125

Gly Ser Val Ile Cys Pro Ser Gln Glu His Ile Asn Ala Ala Thr Thr
    130                 135                 140

Pro Gln Gln Lys Gln Ile Leu Gly Asn Leu Thr Leu Thr Asp Met Gly
145                 150                 155                 160

<210>  45
<211>  355
<212>  PRT
<213>  Aspergillus ficuum

<400>  45

Cys Leu Pro Pro Thr Pro Gly Val Val Gly Pro Tyr Pro Tyr Asp Leu
1               5                   10                  15

Ser Lys Ile Pro Lys Gly Phe Tyr Glu Gly Cys Thr Pro Ser Glu Gly
            20                  25                  30

Pro Thr Glu Val Phe Lys Val Asn Ser Ser Ser Arg Tyr Ala Ser Phe
        35                  40                  45

Asp Phe Ile Ser Met Ala Gly Ser Thr Ser Leu Val Phe Ser Ile Asp
    50                  55                  60

Glu His Pro Met Tyr Val Tyr Ala Val Asp Gly Arg Tyr Val Glu Pro
65                  70                  75                  80

Leu Leu Val Glu Ala Val Thr Val Pro Val Ala Ser Arg Tyr Ser Val
                85                  90                  95

Met Val Pro Leu Lys Ser Glu Asp Gln Ala Gly Asp Tyr Thr Ile Arg
            100                 105                 110

Val Ala Asn Asn Tyr Ala Asn Gln Leu Ile Asn Gly Thr Ala Val Leu
        115                 120                 125

Ser Tyr Asp Thr Ala Thr Pro Lys Gln Ile Gly Thr Ser Gln Pro Tyr
    130                 135                 140

Ile Asn Glu Ala Gly Ala Asn Ala Thr Ala Ser Thr Val Ile Leu Asn
145                 150                 155                 160

Glu Thr Asp Val Ile Pro Phe Pro Val Val Ala Pro Ala Lys Lys Ala
                165                 170                 175

Asp Arg Thr Tyr Ile Leu Asn Val Glu Asn Ala Asn Ser Ser Tyr Thr
            180                 185                 190

Trp Thr Leu Gly Asn Gln Tyr Pro Val Ser Asn Glu Glu Leu Arg Pro
        195                 200                 205

Pro Val Leu Phe Asn Leu Ser Ser Ile Ser Gln Ala Tyr Ser Ala Met
    210                 215                 220

Thr Glu Tyr Gly Thr Trp Val Asp Leu Ile Ile Asn Ile Thr Thr Ser
225                 230                 235                 240

Gly Gln Pro Gln His Pro Ile His Lys His Ser Asn Lys Tyr Phe Val
                245                 250                 255

Ile Gly Thr Gly Asn Gln Pro Phe Ile Trp Ser Ser Val Glu Glu Ala
            260                 265                 270

Met Lys Asp Ile Pro Glu Asn Phe Asn Phe Glu Asn Pro Gln Met Arg
        275                 280                 285

Asp Thr Phe Tyr Ser Pro Ser Ser Ser Thr Gly Pro Ser Trp Leu Ala
    290                 295                 300

Met Arg Tyr His Ile Val Asn Pro Gly Pro Phe Leu Leu His Cys His
305                 310                 315                 320

Leu Gln Met His His Val Gly Gly Leu Ala Leu Ala Leu Leu Asp Gly
                325                 330                 335

Val Asp Ala Trp Pro Thr Asp Ile Pro Glu Gly Tyr Arg Leu Pro Val
            340                 345                 350

Met Pro Ile
        355

<210>  46
<211>  10
<212>  PRT
<213>  Aspergillus

<400>  46

Ala Val Val Gln Phe Gln Leu Asp Leu Thr

1                   5                   10

<210>  47
<211>  11
<212>  PRT
<213>  Aspergillus

<400>  47

Ser Glu Asp Gln Ala Gly Asp Tyr Thr Ile Arg
1               5                   10

<210>  48
<211>  30
<212>  PRT
<213>  Aspergillus

<220>
<221>  misc_feature
<222>  (19)..(19)
<223>  Xaa can be any naturally occurring amino acid

<220>
<221>  misc_feature
<222>  (21)..(21)
<223>  Xaa can be any naturally occurring amino acid

<220>
<221>  misc_feature
<222>  (29)..(29)
<223>  Xaa can be any naturally occurring amino acid

<400>  48

Ala Val Val Gln Phe Gln Leu Asp Leu Thr Tyr Glu Asp Val Ser Val
1               5                   10                  15

Ala Gly Xaa Val Xaa Lys Ala Ile Val Leu Asn Gly Xaa Ile
            20                  25                  30

<210>  49
<211>  18
<212>  PRT
<213>  Aspergillus

<220>
<221>  misc_feature
<222>  (11)..(12)
<223>  Xaa can be any naturally occurring amino acid

<400>  49

Tyr Glu Asp Val Ser Val Ala Gly Lys Val Xaa Xaa Ala Ile Val Leu
1               5                   10                  15

Asn Gly

<210>  50
<211>  14
<212>  PRT

<213>  Aspergillus

<220>
<221>  misc_feature
<222>  (5)..(5)
<223>  Xaa can be any naturally occurring amino acid

<400>  50

Ala Ser Gln Tyr Xaa Ser Tyr Ile Tyr His Ser His Thr Arg
1               5                   10

<210>  51
<211>  21
<212>  DNA
<213>  Aspergillus nidulans

<400>  51
gaagtggaaa ggctggtgtg c                                                21

<210>  52
<211>  11
<212>  DNA
<213>  Artificial Sequence

<220>
<223>  Sfi I adaptor

<400>  52
gttggccttt t                                                           11

## Description

### Field of the invention

The present invention relates to a new isolated nucleic acid sequence comprising a gene that encodes a new fungal oxidase enzyme, and nucleic acid fragments thereof.

It also relates to said new fungal oxidase isolated and purified from *Aspergillus ficuum,* its amino acid sequence as shown in SEQ ID NO 40 or 41, and functional equivalents or derivatives thereof.

The present invention also relates to constructs, vectors and hosts cells comprising a nucleic acid molecule of the invention, as well as methods for producing an oxidase of the invention.

The present invention also relates to the use of an oxidase of the invention in industrial processes.

### Background of the invention

Oxidases are enzymes that catalyze many kinds of biological oxidations.

Among these oxidases, for example, laccases (also referred to as polyphenol oxidases ; EC 1.10.3.1. ; benzenediol:oxygen oxidoreductases) are multi-copper containing enzymes that catalyze the oxidation of a variety of phenolic compounds with concomitant reduction of O₂ to H₂O. These polyphenol oxidases are widely spread and produced by a wide variety of (1) fungi including (a) ascomycetes such as *Aspergillus, Neurospora* or *Podospora,* (b) the deuteromycete *Botrytis,* (c) basidiomycetes such as *Collybia, Fomes, Lentinus, Pleurotus, Trametes, Phlebia* or *Pycnoporus,* (d) perfect forms of *Rhizoctonia,* but also by (2) plants such as *Rhus vernicifera, Liriodendron tulipifera, Nicotiana tabacum* or *Acer pseudoplatanus,* and by (3) bacteria such as *Azospirillum lipoferum.* They are also widespread in bacteria (Alexandre and Zhulin, 2000, Tibtech 18: 41).

Taking into account the biodiversity of their producers, oxidases and laccases exhibit a wide range of substrate specificities with different abilities to oxidize phenolic substrates. Thus, laccases are involved in pigmentation, fruiting body formation, pathogenicity and lignin degradation and biosynthesis.

Because of this substrate specificity, oxidases and laccases show potential in industrial applications (pulp and paper processing, dye transfer inhibition in detergents or phenol polymerization), in environmental applications (environmental pollutants detoxification or waste water treatment), in food application (baking, brewing, prevention of wine discoloration, color enhancement of tea based foodstuff, deoxygenation of food items, or juice manufacture) and in pharmaceutical applications (transformations of steroid and antibiotics) (see for examples: Sariaslani, 1989, Critic. Rev. Biotechnol. 9:171; Potus et al., 1999, Industries des céréales, 115:3; Lopez et al., 2002, J. Biotechnol., 99:249; Duran et al., 2002, Enz. Microbial Technol., 31:907; Minussi et al., 2002, Trends Food Sci. Technol., 13:205; Biotechnology in the pulp and paper industry, 2002, Viikari et Lantto eds, Elsevier).

Depending on their origins, fungal laccases have different temperature and pH optima, different redox potential and substrate specificities (Xu F. & al., 1996, Biochim Biophys Acta. 1292, p.303). A large number of fungal laccases have been isolated and most of their corresponding genes have been cloned. Similarities and strong identities values found between their amino acid sequences show that closely related sequences belongs to organisms which are members of the same phylogenetic group. These values are sometimes higher between enzymes from different species of the same genus than between different laccases produced by the same species (Eggert et al., 1998, Appl Environ Microbiol. 64: 1766).

Depending on their wide range of substrate specificity, oxidases and laccases have great commercial potential and the ability to express these enzymes at very high level is critical for commercial purposes. Attempts to express laccase genes in heterologous fungal systems frequently gave very low yields. Thus, the expression of *Phlebia radiata* laccase in *Trichoderma reesei* gave only 20 mg per liter of active enzyme (Saloheimo et al., Bio/Technology 1991, 9: 987), while expression of *Coprinus cinereus* Lcc1 laccase in *Aspergillus oryzae* gave 8 to 135 mg per liter (Yaver et al., 1999, lcc1. Appl Environ Microbiol., 65(11):4943-8.) and that of *Myceliophtora thermophila* in *Aspergillus oryzae* gave 11 to 19 mg per liter (Berka et al., 1997, Appl Environ Microbiol. 63: 3151). The laccase of *Pycnoporus cinnabarinus* has been expressed at a level of about 80 mg per liter in *Aspergillus* (Sigoillot C. et al., 2004, Appl. Microbiol. Biotechnol. 64: 346).

At the present time there is still a need for new oxidases that can be used in the different applications described herein.

Furthermore the expression of their corresponding genes at high level in industrial hosts such as *Aspergillus* is of great interest.

### Summary of the invention

The present invention relates to the isolation and characterization of a new gene encoding an *Aspergillus ficuum* oxidase.

A new gene encoding an oxidizing enzyme according to the invention has been isolated from A. *ficuum* and is 1,923 base pairs long with two introns and an open reading frame corresponding to 596 amino acids.

An aspect of the invention relates to a nucleic acid molecule comprising or consisting of SEQ ID NO 1, 2, 3, 4, 5, 6, 7, 8, 9, 10, or 11 encoding an *A. ficuum* oxidase.

Another aspect of the invention relates to a nucleic acid molecule encoding a polypeptide of the present invention.

Another aspect of the invention relates to a polynucleotide selected from the group consisting of:
- a nucleic acid molecule comprising a nucleotide sequence having at least 60%, advantageously at least 70%, more advantageously at least 80%, preferably at least 85%, more preferably at least 90% and even more preferably at least 95% identity with any of SEQ ID NO 1 to 39 the complementary form and RNA form thereof,
- any fragments thereof encoding a protein having an oxidase activity, the complementary form and RNA form thereof, and,
- any fragments thereof of at least 15 nucleotides, preferably of at least 20 nucleotides, or more preferably of at least 25 nucleotides, the complementary form and RNA form thereof.

Another aspect on the invention relates to a protein having an oxidase activity, of about 85 kDa as shown by polyacrylamide gel electrophoresis and Coomassie blue staining.

Another aspect on the invention relates to a protein having an oxidase activity with a molecular weight of about 70 kDa after deglycosylation with PNGaseF.

The invention also relates to an isolated oxidase polypeptide, the amino acid sequence of which comprises or consists of SEQ ID NO 40 or 41, and any fragments thereof having an oxidase activity.

Another aspect of the invention relates to an isolated polypeptide having an oxidase activity, selected from the group consisting of an amino acid sequence having at least 70%, advantageously at least 80%, preferably at least 85%, more preferably at least 90% and even more preferably at least 95% identity with SEQ ID NO 40 or 41.

Another aspect on the invention relates to transformed cells, e.g. *A. nidulans 2024,* comprising a gene according to the invention expressed under the control of its own promoter.

Another aspect on the invention relates to transformed cells, e.g. *A. nidulans* or *A. ficuum,* comprising a gene according to the invention expressed under the control of the gpdA promoter of *A. nidulans* (glyceraldehyde-phosphate-dehydrogenase promoter), resulting in a several-fold increase of the expression of said gene.

Another object of the invention relates to the use of an oxidase according to the present invention in food and non-food industrial applications, where oxidation of phenolics is required, for example its use in a baking process or its use as colour enhancer, e.g. in tea.

Another object of the invention relates to a bread improving composition comprising an oxidase polypeptide of the invention.

### BRIEF DESCRIPTION OF THE DRAWINGS

Figure 1 represents a SDS-PAGE and zymogram analysis of the purified enzyme with oxidase activity from *Aspergillus ficuum* (maintained as a deposit with DSMZ under the accession number DSM932).

Figure 2 represents the enzyme activity in function of the pH.

Figure 3 represents the nucleotide sequence of the gene coding for the enzyme with oxidase activity from *Aspergillus ficuum* (DSM932), and the corresponding amino acid sequence.

Figure 4 represents the absorbance spectra of tea solutions treated or not with an oxidase according to the invention.

Figure 5 represents the effect of increasing amounts of an oxidase isolated from *Aspergillus ficuum* (DSM932) according to the invention on the volume of bread.

### DETAILED DESCRIPTION OF THE INVENTION

### Oxidase encoding gene.

The present invention provides an isolated nucleic acid molecule encoding an oxidase.

The nucleic acid molecule consisting of SEQ ID NO 1 has been isolated from *A. ficuum* deposited with DSMZ under the accession number DSM932 (referred to as *A. ficuum* DSM932).

Allelic and species variants are also contemplated and are referred to as homologues or homologous sequences.

In the context of the present invention, a homologue or homologous sequence is a nucleotide or an amino acid sequence having at least 60%, advantageously at least 70%, more advantageously at least 80%, preferably at least 90%, and more preferably at least 95%, 96%, 97%, 98% or 99% homology (or identity) with any of SEQ ID NO 1 to 50.

It is meant by "homology" or "identity" the value in percentage given when comparing two sequences. Said comparison can be performed using any available software, for example the Clustalw program and the default parameters values (e.g. available at http://www.ebi.ac.uk/clustalw).

In the context of the present invention the terms "polynucleotide" or "nucleic acid molecule" refer to single-stranded or double stranded molecules and include DNA molecules (e.g. cDNA or genomic DNA), RNA molecules (e.g. mRNA) and analogs wherein nucleotides have been replaced by nucleotide analogs or derivatives.

A nucleic acid consisting of or comprising any of SEQ ID NO 1 to 39 or any homologue, its complementary form (or complementary strand), or its RNA form can be isolated from different micro-organisms producing an oxidase according to the invention.

Said micro-organisms can be bacteria or fungi, including yeasts, and can be more specifically other *Aspergillus* species.

Established standards methods can be used to isolate a homologous sequence of the invention.

Examples of such methods include the construction of a gene library from the genomic DNA of the micro-organisms in a suitable vector, followed by screening of this library by direct expression of said homologous sequence.

Another method comprises a hybridization step with e.g. a fragment of at least 15 nucleotides, preferably at least 20 nucleotides and more preferably at least 50 nucleotides of a nucleic acid molecule of the invention, e.g. a fragment of SEQ ID NO 37, 38 or 39.

Other methods include the amplification of said homologous sequence by molecular techniques, for instance PCR techniques, using oligonucleotide primers that are designed from a nucleic acid sequence of the invention.

Furthermore, there are established methods for obtaining in a relatively short period of time thousands of mutated sequences together with assessed enzymatic activities of their corresponding polypeptide sequences. These methods include e.g. random mutagenesis, high-throughput screening, etc., and are frequently used to demonstrate the eventual effects of single or multiple mutations.

Such mutations (addition(s), deletion(s) and/or substitution(s)) can be silent or not, can be made inside or outside the regions critical to the function of the molecule and still result in an active protein having an oxidase activity more or less similar to the oxidase activity of a polypeptide of SEQ ID NO 40 or 41, i.e. of at least 70%, advantageously of at least 80%, preferably of at least 90%, or more preferably of at least 95% (also referred to as functional equivalents).

Alternatively a nucleic acid molecule of the invention may be prepared synthetically by methods known in the art. A nucleic acid molecule of the invention may include oligonucleotide analogs or derivatives (e.g. inosine or phosphorothioate nucleotides, etc.) so it has, for example, altered base-pairing abilities or increased resistance to nucleases.

A nucleic acid molecule of the invention may or may not include introns interrupting the coding sequence.

A nucleic acid molecule of the invention may be of mixed genomic, synthetic and/or cDNA origin, prepared by methods known in the art comprising the step of ligating fragments from different origins.

A preferred isolated and purified nucleotide sequence of the invention corresponds to SEQ ID NO 1, 2, 3, 4, 5, 6, 7, 8, 9, 10, or 11 or a fragment thereof that encodes a peptide having an oxidase activity.

A fragment of said sequence SEQ ID NO 1, 2, 3, 4, 5, 6, 7, 8, 9, 10, or 11 has preferably more than 600 nucleotides, more preferably more than 900 nucleotides or even more preferably more than 1200 nucleotides and encodes a protein characterized by a oxidase activity similar to the oxidase activity of the complete amino acid sequence of SEQ ID NO 40 or 41 (also referred to as a functional equivalent).

Preferably, said functional equivalent has an oxidase enzymatic activity of more than 70%, or more than 80% of the initial oxidase activity of the complete enzyme defined by its amino acid sequence of SEQ ID NO 40 or 41, and preferably has an oxidase activity of at least 90% compared to the one having the amino acid sequence of SEQ ID NO 40 or 41.

A polynucleotide of the invention is selected from the group consisting of:
- (a) a nucleic acid molecule comprising a nucleotide sequence having at least 60%, advantageously at least 70%, more advantageously at least 80%, preferably at least 85%, more preferably at least 90% and even more preferably at least 95%, 96%, 97%, 98% or 99% identity with any of SEQ ID NO 1 to 39, the complementary form and RNA form thereof,
- (b) any fragments of a nucleic acid molecule of group (a) encoding a protein having an oxidase activity, the complementary form and RNA form thereof, and
- any fragments of a nucleic acid molecule of group (a) or (b) of at least 15 nucleotides, preferably of at least 20, 25, or 30 nucleotides, or more preferably of at least 50 nucleotides, the complementary form and RNA form thereof.

In other words, a nucleic acid molecule according to the invention may comprise or consist of:
- a nucleotide sequence of any of SEQ ID NO 1 to 39, its complementary form or RNA form,
- a nucleotide sequence having at least 60%, advantageously at least 70%, more advantageously at least 80%, preferably at least 85%, more preferably at least 90% and even more preferably at least 95%, 96%, 97%, 98% or 99% identity with any of SEQ ID NO 1 to 39, or with the complementary form or RNA form thereof,
- a fragment of any of SEQ ID NO 1 to 11 or of any of their homologues, or of any of their complementary form or RNA form, wherein said fragment encodes a protein having an oxidase activity, or
- a fragment of at least 15 nucleotides, preferably at least 20 nucleotides, more preferably of at least 25 nucleotides, of any of SEQ ID NO 1 to 39 or of any of their homologues, or of any of their complementary form or RNA form.

Said fragments of any of SEQ ID NO 1 to 11 or of any of their homologues, or of any of their complementary form or RNA form, encoding a protein having an oxidase activity, consist preferably of at least 600 nucleotides, preferably of at least 900 nucleotides, or more preferably of at least 1200 nucleotides.

A nucleic acid molecule according to the invention comprising or consisting of a fragment of at least 15 nucleotides, preferably of at least 20, 25, or 30 nucleotides, more preferably of at least 50 nucleotides, of any of SEQ ID NO 1 to 39 or of any of their homologues, or of any of their complementary form or RNA form, can be used for example for detection or identification purposes, as a primer or a probe.

### Oxidase Protein.

Also provided is a protein having an oxidase activity, of about 85 kDa as shown by polyacrylamide gel electrophoresis and Coomassie blue staining.

A protein of the invention, having an oxidase activity, has a molecular weight of about 70 kDa after deglycosylation with PNGaseF.

Advantageously the unglycosylated form of the isolated and purified amino acid sequence according to the invention has a molecular weight comprised between about 60 and about 70 kDa, preferably about 63 kDa or about 65.5 kDa.

An isolated oxidase of the invention consists of a polypeptide encoded by a nucleic acid of the invention.

An isolated oxidase polypeptide of the invention may comprise or consist of:
- an amino acid sequence of any of SEQ ID NO 40 to 50,
- a fragment of at least 100 amino acids of SEQ ID NO 40, 41 or 45, or
- an amino acid sequence presenting at least 60% identity with the amino acid sequence of any of SEQ ID 40 to 50, or with any fragments of at least 100 amino acids of said SEQ ID NO 40, 41 or 45.

More specifically, an isolated oxidase of the invention consists of a polypeptide encoded by a nucleic acid molecule comprising or consisting of:
- Any of SEQ ID NO 1 to 39,
- a nucleotide sequence having at least 60%, advantageously at least 70%, more advantageously at least 80%, preferably at least 85%, more preferably at least 90% and even more preferably at least 95%, 96%, 97%, 98% or 99% identity with any of SEQ ID NO 1 to 39 (also referred to as homologues), or
- any fragments of any of SEQ ID NO 1 to 11, or of their homologues, encoding a protein having an oxidase activity.

An isolated oxidase polypeptide of the invention comprises or consists of the amino acid sequence of any of SEQ ID NO 40 to 50, or any fragments thereof that have retained an oxidase activity.

An isolated oxidase polypeptide of the invention comprises or consists of an amino acid sequence presenting at least 60%, preferably at least 70%, 80% or 85%, more preferably at least 90%, or even more preferably at least 95%, 96%, 97%, 98% or 99% homology (or sequence identity) with the amino acid sequence of SEQ ID 40 or 41, or with any fragments of said SEQ ID NO 40 or 41 having an oxidase activity.

A preferred fragment of an oxidase polypeptide of the invention, in particular a preferred fragment of SEQ ID NO 40 or 41, consists or comprises an amino acid sequence of at least 100 amino acids, preferably of at least 200, more preferably of at least 300 amino acids, and even more preferably of at least 400 amino acids.

A preferred fragment of an oxidase polypeptide of the invention has at least 70%, advantageously at least 80%, more advantageously at least 85%, preferably at least 90%, or more preferably at least 95%, 96%, 97%, 98% or 99% of the oxidase activity of an oxidase polypeptide defined by an amino acid sequence of SEQ ID NO 40 or 41.

A preferred fragment of an oxidase polypeptide of the invention, in particular a preferred fragment of SEQ ID NO 40 or 41, consists or comprises an amino acid sequence of at least 100 amino acids, preferably of at least 200, more preferably of at least 300 amino acids, and even more preferably of at least 400 amino acids, and has at least 70%, advantageously at least 80%, more advantageously at least 85%, preferably at least 90%, or more preferably at least 95%, 96%, 97%, 98% or 99% of the oxidase activity of an oxidase polypeptide defined by an amino acid sequence of SEQ ID NO 40 or 41.

Indeed, an isolated oxidase polypeptide of the invention consisting of or comprising an amino acid sequence of any of SEQ ID NO 40 to 50 can be deleted partially while maintaining its enzymatic activity. Said enzymatic activity can be measured by methods well known in the art.

A protein fragment according to the invention can also be prepared by recombinant techniques.

An isolated oxidase polypeptide according to the invention may also result from the substitution, deletion and/or insertion of one or more amino acids in an amino acid sequence of any of SEQ ID NO 40 to 50.

Said substitution can be conservative, which means that an amino acid is replaced with an amino acid having a similar side chain without affecting significantly the enzymatic activity of said polypeptide compared to an oxidase polypeptide consisting of the amino acid sequence of SEQ ID NO 40 or 41. These families are known in the art and include amino acids with basic side chains (e.g. lysine, arginine and histidine), acidic side chains (e.g. aspartic acid, glutamic acid), uncharged polar side chains (e.g. glycine, asparagine, glutamine, serine, threonine, tyrosine, cysteine), non-polar side chains (e.g. alanine, valine, leucine, isoleucine, proline, phenylalanine, methionine, tryptophan), beta-branched side chains (threonine, valine, isoleucine) and aromatic side chains (e.g. tyrosine, phenylalanine, tryptophan, histidine).

Moreover, said substitution, deletion or insertion may concern non-essential amino acid, also resulting in no significant alteration of the oxidase activity of said polypeptide in comparison with an oxidase polypeptide consisting of the amino acid sequence of SEQ ID NO 40 or 41.

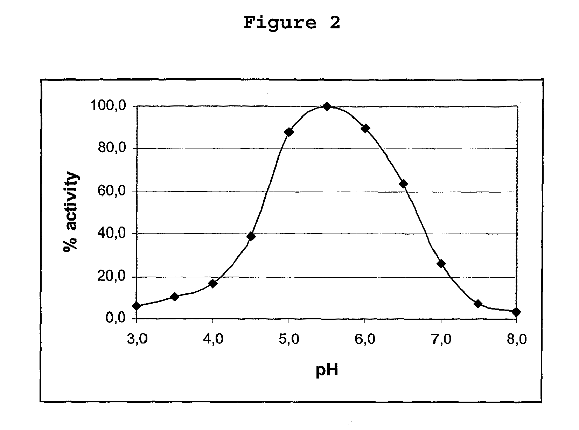

An isolated and purified oxidase enzyme according to the invention is also characterized by an optimum pH around 5.5. More generally the maximum activity is comprised between a pH of about 5 and a pH of about 6.5 (see figure 2).

An isolated oxidase polypeptide according to the invention can also be characterized by the fact that it can use, among others, N,N-dimethyl-p-phenylenediamine and 2,2'-azinobis(3-ethylbenzthiazoline-6-sulfonic acid) as substrates.

Therefore an isolated oxidase polypeptide according to the invention can also be referred to as a laccase.

### Expression and/or purification of the enzyme

Another aspect of the present invention is related to a recombinant nucleotide sequence comprising, operably linked to a nucleotide sequence according to the invention, one or more adjacent regulatory sequence(s). Said adjacent regulatory sequence(s) are preferably originating from homologous micro-organisms.

However said adjacent regulatory sequences may also be originating from heterologous micro-organisms.

Said adjacent regulatory sequences are specific sequences such as promoters, enhancers, secretion signal sequences and/or terminators.

Preferred adjacent regulatory sequences of the invention are capable of directing the overexpression of a nucleotide sequence of the invention in a recombinant host cell. A preferred adjacent regulatory sequence according to the invention is the constitutive gpdA (glyceraldehyde -3-phosphate dehydrogenase) promoter of *Aspergillus nidulans.*

Another aspect of the invention is related to a vector comprising a nucleic acid molecule of the invention, possibly operably linked to one or more adjacent regulatory sequence(s) originating from homologous or from heterologous micro-organisms.

In the present context "vector" is defined as any biochemical construct which may be used for the introduction of a nucleotide sequence (by transduction, tranfection, transformation, infection, conjugation, etc.) into a cell.

Advantageously, a vector according to the invention is selected from the group consisting of plasmids (including replicative and integrative plasmids), viruses, phagemids, chromosomes, transposons, liposomes, cationic vesicles, or a mixture thereof. Said vector may already comprise one or more adjacent regulatory sequence(s), allowing the expression of said nucleic acid molecule and its transcription into a polypeptide of the invention. Preferably said vector is a plasmid.

The present invention is also related to a transformed host cell, or recombinant host cell, containing one or more of the nucleotide sequences and/or vectors according to the invention.

In the present context, a "transformed host cell" or "recombinant cell", also referred to as "transformant", is a cell having incorporated one or more of the nucleotide sequences and/or vectors according to the invention. The transformed host cell may be a cell in which said vector(s) and/or said nucleotide sequence(s) is/are introduced by means of genetic transformation, preferably by means of homologous recombination, or by any other well known methods used for obtaining a recombinant organism.

Said host cell used for the transformation, may or may not already have (a) nucleotide sequence(s) and/or vector(s) of the invention. Both prokaryotic and eukaryotic cells are included, e.g. bacteria, fungi, yeast, etc.

Preferred host cells are A. *nidulans,* in particular *A. nidulans* 2024, *A. niger,* more specifically A. *niger* N402 or *A. ficuum,* more particularly A. *ficuum* DSM 932.

Said host cell may also be the original cell, e.g. *A. ficuum,* containing already nucleotide sequences of the present invention, and genetically modified to over-express, or express more efficiently, an oxidase polypeptide of the invention (better pH or temperature profile, higher extracellular expression, etc.).

A transformed host cell of the invention may have integrated into its genome an isolated nucleic acid molecule according to the present invention and/or may contain (an) episomal vector(s) comprising an isolated nucleic acid molecule of the invention.

A preferred transformed host cell of the invention is capable of over-expressing (i.e. higher expression than the expression observed in the original or wild-type microorganism) (a) nucleotide sequence(s) and/or vector(s) of the invention, advantageously allowing a high production of polypeptide encoded by said nucleotide sequence(s) and/or said vector(s).

Preferably, said recombinant host cell contains regulatory sequences adjacent to a nucleic acid molecule according to the invention, that are capable of directing the overexpression of said nucleic acid molecule.

An overexpression of an oxidase of the invention may also result from an increasing number of copies of nucleic acid sequences according to the invention in said recombinant host cell.

For an optimal expression of an oxidase according to the invention, the original production species, e.g. *A. ficuum,* and/or a suitable transformed host cell, e.g. transformed *A. niger* or transformed A. *nidulans,* are in a suitable growth medium and/or expression medium. Some examples of the optimal conditions, such as culture media, temperature and pH conditions, etc., are described in the examples section.

According to the present invention, a polypeptide of the invention with an oxidase activity may be obtained by first culturing the strain in/on a medium suitable for expressing said oxidase, and then may be recovered from the medium by conventional methods including but not limited to centrifugation, microfiltration, ultra-filtration, spray-drying, evaporation or precipitation. Said oxidase according to the invention may be further purified using for example electrophoretic procedures, extraction, or a variety of chromatographic procedures, such as ion exchange chromatography, gel filtration chromatography, affinity chromatography, etc. All these techniques are described in the scientific literature and are well known techniques.

Said oxidase can be extra-cellular or intracellular expressed and/or secreted by a micro-organism producing said oxidase or by a recombinant host according to the invention.

Said polypeptide of the invention may be expressed in a modified form, such as a fusion protein, and may include one or more secretion signals and/or one or more additional heterologous functional regions. Said regions may consist of particularly charged amino acids added to the Nₜ of said polypeptide to improve stability and persistence in the host cell, during purification of during subsequent handling and storage. They may also consist of short peptides added to said polypeptide of the invention to facilitate purification.

### Different applications of an oxidase of the invention

The oxidase enzymes according to the invention may be used in different kinds of industries.

A polypeptide with oxidase activity according to the present invention, further purified or not purified, is particularly suited as a bread improving agent. Bread improving agents or bread improving compositions are products that are able to improve and/or increase texture, flavour, anti-staling effects, softness, crumb softness upon storage, freshness, dough machinability and/or volume of a dough and/or of a final baked product.

A polypeptide with oxidase activity according to the invention is preferably used to improve the dough handling and/or increase the specific volume of the final baked product. A polypeptide with oxidase activity according to the invention is advantageously used in a bread improver formula or bread improving composition.

The present invention also relates to a bread improving composition comprising an oxidase polypeptide of the invention.

The term "baked product" includes any product prepared from a dough and obtained after baking of the dough, and includes in particular yeast raised baked products.

Dough is obtained from any type of flour or meal (e.g. based on wheat, rye, barley, oat, or maize). Preferably, dough is prepared with wheat and/or with mixes including wheat.

A bread improving composition according to the invention may also comprised other bread-improving agents such as, but not limited to enzymes, emulsifiers, oxidants, milk powder, fats, sugars, amino acids and/or proteins (gluten, cellulose binding site).

Examples of such enzymes include, but are not restricted to, alpha-amylases, beta-amylases, maltogenic amylases, xylanases, proteases, glucose oxidases, oxido-reductases, glucanases, cellulases, transglutaminases, isomerases, lipases, phospholipases, pectinases, etc.

A preferred bread improving composition according to the invention comprises an oxidase polypeptide of the invention and an alpha-amylase, preferably an alpha-amylase from *Aspergillus oryzae.*

In another aspect of the present invention an oxidase polypeptide of the invention is particularly suited for the improvement or enhancement of the color of tea based foodstuffs.

An oxidase polypeptide of the invention may be used in food applications such as baking, pastry, cakes, brewing, prevention of wine discoloration, deoxygenation of food items, juice manufacturing, etc., or in feed applications.

In another aspect of the present invention, an oxidase polypeptide of the invention may be used in industrial applications such as pulp and paper processing, dye transfer inhibition in detergents or phenol polymerization, etc., in environmental applications such as environmental pollutants detoxification, waste water treatment, etc., or in pharmaceuticals applications (transformations of steroids and antibiotics, etc.).

The effect of an oxidase polypeptide of the invention may be further improved by adding other enzymes. Such enzymes may belong, but are not restricted, to hydrolytic enzymes families such as glucanase, proteases, cellulases, hemicellulases, and pectinases. Other enzymes are transglutaminases, oxido-reductases, isomerases, etc.

Depending on the application, an oxidase polypeptide of the invention may be used under several forms. Micro-organisms (recombinant or not) expressing an oxidase polypeptide of the invention, such as yeasts, fungi, archea bacteria or bacteria, may be used directly in the process.

An oxidase polypeptide of the invention may be used as a cell extract, a cell-free extract (i.e. portions of the host cell that has been submitted to one or more disruption, centrifugation and/or extraction steps) and/or as a purified protein.

One or more of said forms may be used in combination with one or more enzymes under any of the above-described forms.

Said whole cells, cell extracts, cell-free extracts or said purified oxidase polypeptide of the invention may be immobilized by any conventional means on a solid support for instance to allow protection of the oxidase polypeptide of the invention, or to allow continuous hydrolysis of a substrate and/or to allow recycling of the enzymatic preparation.

Said cells, cell extracts (including crude and partially purified extracts), cell-free extracts and/or said purified oxidase polypeptide of the invention may be mixed with different ingredients, e.g. in the form of a dry powder or a granulate, in particular a non-dusting granulate, or in a form of a liquid, for example with stabilizers such as polyols, sugars, organic acids, sugar alcohols according to well-established methods.

The invention is described in further details in the following examples, which are intended for illustration purposes only, and should not be construed as limiting the scope of the invention in any way.

### EXAMPLES

### MATERIALS AND METHODS

### EXAMPLE 1: Strains and media.

The bacterial strains used were the E. coli strains RR1ΔM15 (F'lacIQ lacZΔM15 hsdS20 supE44 ara-14 proA2 rspL20(strR) lacY1 galK2 xyl-5 mtl-1) and MC1061 (hsdR mcrB araD139 Δ (araABC-leu) 7697 Δ lacX74 galU galK rpsL thi). Fungal strains were *Aspergillus ficuum* DSM932, *Aspergillus nidulans* 2024 (biA1 argB3) and *Aspergillus niger* N402 (cspAl derivate of the ATCC strain 9029).

Bacteria were grown at 37°C in LB (0.5% yeast extract, 1% Bacto peptone, 1% NaCl) or TB medium (Terrific Broth, Gibco BRL Life Technologies Inc., Gaithersburg, MD) and fungi in *Aspergillus* minimal medium (Ponteverco & al, 1953, Adv. Genet.,5:.141) at 28°C and 250 rpm.

### EXAMPLE 2: Fermentations

*Aspergillus* strains were cultivated in 151 Biostat E fermentors (B. Braun Biotech - working volume 101). The culture medium composition was the following: Maldex 15: 40 g/l; Salt solution: 50ml/l; Trace elements solution: 1ml/l; CuSO4 solution (1.6g/l): 1ml/l. The salt solution contained 120g/l NaNO3, 10.40g/l KCl, 10.40 g/l MgSO4. 7H2O and 30.40g/l KH2PO4. The Trace elements solution (pH 6.5) contained 22g/l ZnSO4. 7H2O, 11g/l H3BO3, 4.1g/l MnCl2. 2H2O, 5g/l FeSO4. 7H20, 1.7g/l CoCl2. 6H2O, 1.6g/l CuSO4. 5H2O, 1.5g/l Na2MoO4. 2H20 and 50g/l ethylenedinitrilotetraacetic acid disodium salt dihydrate. 1 mg/l biotine was added after sterilization.

The initial pH of the fermentation was 6.0 and the temperature was fixed at 30°C. The duration of the fermentation was around 60 to 70 hours.

### EXAMPLE 3: Isolation of genomic DNA.

The preparation of genomic DNA from mycelium of *A. ficuum* was based on the protocol of Blin and Stafford (Nucl. Ac. Res.,1976, 3:.2303).

The mycelium was washed with 100% ethanol, dried in a vacuum desiccator and ground to powder under liquid nitrogen. The powder was resuspended in extraction buffer (50 mM Tris-HCl pH 7.4, 10 mM MgCl2, 50 mM NaCl, 1% SDS) and incubated at 55 °C for 15 min.

Phenol/chloroform (1/1 v/v) was added and the solution was placed on a rocking platform at room temperature for 30 min. The mixture was centrifuged 15 min at 3,000 rpm. The DNA phase was extracted again with phenol/chloroform and then with chloroform.

The DNA was precipitated with the addition of 0.1 volume of 3 M NaAc and 0.5 volume of isopropanol at room temperature.

After centrifugation the pellet was washed with 70% ethanol, briefly dried and dissolved in TE-buffer (10 mM Tris-HCl pH 8.O, 1 mM EDTA).

To remove residual RNA, 100 *µ*g DNAse-free RNAse A was added and the solution was incubated for 30 min at 37°C. SDS to a final concentration of 0.5% and predigested proteinase K to a final concentration of 50 *µ*g/ml were added to the DNA solution for 1 hour at 50°C.

Extraction was performed once with an equal volume of phenol/chloroform and once with chloroform. The DNA was precipitated again, centrifuged, washed with 70% ethanol and redissolved in TE-buffer.

### EXAMPLE 4: PCR amplification on genomic DNA.

Based on the amino-terminal sequence (SEQ ID NO 46) and on the sequence of internal peptide (1) (SEQ ID NO 47), a set of degenerated oligonucleotides primers was synthesized.

NH2-terminal sequence (SEQ ID NO 46):
A V V Q F Q L D L T

forward PCR-primer (SEQ ID NO 37):
5' GTI GTI CAG TTT CAG YTI GAT YTI AC 3'

internal sequence 1 (SEQ ID NO 47):
S E D Q A G D Y T I

R reverse PCR-primer (SEQ ID NO 38)
3' CTY CTR GTY CGI CCI CTG ATG TGI TA 5'

These two primers were used for PCR amplification. PCR was performed on 100 ng genomic DNA of *A. ficuum* DSM932.

The DNA was suspended in 100 *µ*l of thermophilic buffer (50 mM KCl, 10 mM Tris-HCl pH 9.0, 0.1% Triton X-100; Promega Corporation, Madison, WI), supplemented with 0.2 mM dNTP, 3 mM MgCl2, 50 pmoles of each primer and 1.5 units of Taq-DNA-polymerase (Promega Corporation).

The temperature scheme for the amplification was as follows: 10 min denaturation at 95°C, hot start at 80°C for the Taq-DNA-polymerase, touch-down for two cycles starting at 95°C for 30 sec and 67°C for 2 min. This was repeated with a gradual decline to 65, 63 and 61°C instead of 67°C. The final cycling parameters were: 95°C 30 sec, 60°C 30 sec and 72°C 1 min (35 cycles). The reactions were carried out in a Biometra "Trio-Thermoblock" thermocycler (Biometra, Göttingen, Germany).

### EXAMPLE 5: Sequence analysis of the PCR products.

The PCR products were cloned in the pUC18 vector (Yanisch-Perron & al, 1985, Gene, 33: 103). This plasmid (purified on a Qiagen column (Qiagen Inc., Chatsworth, CA)) was digested with SmaI (New England Biolabs Inc., Beverly, MA), generating blunt ends.

After extraction with phenol/chloroform, the blunt ends were dephosphorylated with calf intestine alkaline phosphatase (Boehringer) in 50 mM Tris-HCl - 0.1 mM EDTA-buffer (pH 8.5) at 37°C for 30 minutes. Electrophoresis through a 1 % agarose gel was performed and the dephosphorylated vector band was eluted and purified by the Geneclean method (Bio 101, La Jolla, CA).

The PCR reaction mixtures were initially purified on a Qiagen column. The ends of the PCR DNA fragments were filled in with 0.2 mM dNTP using Pfu-DNA-polymerase (Stratagene, La Jolla, CA) and T4-DNA-polymerase (Boehringer) in Pfu-buffer (20 mM Tris-HCl pH 8.75, 10 mM KC1, 10 mM (NH4)2SO4 2 mM MgSO4, 0.1% Triton X-100, 0.1 mg/ml bovine serum albumin; Stratagene) at 37°C for 45 min.

After a new Qiagen purification, the blunt ends of the PCR fragments were phosphorylated at 37°C for 30 min with 0.2 mM rATP using T4-polynucleotide kinase (Amersham, Buckinghamshire, England) in kinase buffer (20 mM Tris-HCl pH 7.5, 10 mM MgCl2, 10 mM β-mercaptoethanol), supplemented with 6% polyethylene glycol 8,000 and 8 mM MgCl2.

After electrophoresis through a 0.8% agarose gel, the 1,150 bp DNA fragments was eluted and purified by the Geneclean method.

The eluted DNA fragments were ligated in the pUC18 vector using T4-DNA-ligase (Boehringer) in ligase buffer (66 mM Tris-HCl pH 7.5, 5 mM MgCl2, 1 mM DTE, 1 mM ATP; Boehringer) at 18°C for 16 hours.

The ligation mixtures were transformed in the E. coli strain RR1ΔM15. The colonies obtained were submitted to a DNA extraction following the Birnboim procedure (Birnboim & al, 1979, Nucl. Ac. Res, 7: 1513). The DNA was analyzed by EcoRI - HindIII digestion and electrophoresis through a 1.2% agarose gel.

The plasmid DNA (Qiagen purified) of a positive clone was sequenced following the ABI Taq DyeDeoxy Terminator Cycle Sequencing protocol (ABI, Foster City, CA). The samples were run on an automated ABI373A sequencing system (ABI). The DNA sequences obtained were converted to ASCII format and transferred to a HIBIO DNASIS software package (Pharmacia LKB Biotechnology, Uppsala, Sweden) for assembly.

### EXAMPLE 6: Construction of a genomic DNA library of A. ficuum DSM932.

Genomic DNA (5 *µ*g) of A. *ficuum* DSM932 was partially digested with the restriction enzyme AluI (7.5 units) for 15 min. The DNA ends obtained were further polished with T4-DNA-polymerase (1 unit) and dNTP nucleotides (final concentration of 100 *µ*M of each) for 5 min at 37°C. The partial digest was then extracted with phenol/chloroform and size-fractionated on a 0.7% agarose gel.

The genomic DNA fragments with a length of 9,000 to 23,000 bp were eluted from the gel by centrifugal filtration (Zhu & al, 1985, Bio/Technology, 9: 1014; membrane type GV, 0.22 *µ*m, Millipore Intertech, Bedford, MA), extracted with phenol/chloroform and concentrated by ethanol precipitation.

Sfi I adaptors (5'-GTTGGCCTTTT) (SEQ ID NO 52) were ligated to the DNA ends. The ligation reaction was performed in PEG buffer (25 mM Tris-HCl pH 7.5, 5 mM MgCl2, 2.5 % (w/v) PEG 8,000, 0.5 mM DTT, 0 .4 mM ATP), with 24 units of T4 DNA ligase and 250 pmoles of phosphorylated Sfi I adaptors in a volume of 50 *µ*l overnight at 12°C.

After extraction with phenol/chloroform, the SfiI - SfiI genomic DNA fragments were purified and separated again on a 0.7% agarose gel. Portions of the gel containing fragments from 10 to 20 kb length were cut out, membrane-eluted and concentrated by ethanol precipitation. These fragments (100 ng) were finally cloned into the SfiI-digested YCp50SfiI-SfiI vector (an E. coli/S. cerevisiae shuttle vector derived from the plasmid YCp50 (Trash & al, 1985, Proc. Nat. Acad. Sci. USA, 82: 4374) bearing an EcoRI - HindIII fragment, that contains the hIFNβ gene flanked by two SfiI sites in inverse direction).

The ligations were performed in a volume of 20 *µ*l at 12°C for 4 hours with 8 units T4 DNA ligase (Pharmacia LKB Biotechnology, Uppsala, Sweden), further extracted with phenol/chloroform and finally electroporated in freshly prepared MC1061 cells.

Bacteria were plated on LB agarose and the resulting colonies were scraped off from the plates in groups of 1,000 clones. The library size was approximately 100,000 clones and the average insert size was ± 16 kb.

### EXAMPLE 7: Screening of the genomic DNA library by colony hybridization.

The 1,150 bp fragment was isolated from the pUC18 vector by EcoRI-HindIII digestion, agarose gel electrophoresis, elution and purification by the Geneclean method.

The fragment was randomly labeled with α32P-dCTP using the procedure of Feinberg and Vogelstein (Anal. Biochem, 1984, 137:.266). 75 ng of the 1,150 bp fragment were boiled for 10 minutes and immediately chilled on ice. The labeling reaction was performed at 37°C for 30 minutes in a total volume of 60 *µ*l using 6 units of Klenow polymerase, dGTP, dATP and dTTP (25 *µ*M of each), a hexanucleotide mixture (Random Primed DNA Labeling Kit from Boehringer) and 45 pmoles α32P-dCTP (Amersham, Buckinghamshire, England). The reaction mixture was then dialyzed for 90 minutes against bidistilled water using a membrane filter (type VS, 0.025 mm; Millipore Intertech, Bedford, MA).

Groups of clones of the genomic DNA library of *Aspergillus ficuum* DSM932 were spread out onto LB plates and overnight incubated at 37°C, resulting in 2,000 to 6,000 colonies per plate. These colonies were submitted to colony lifting on nylon membranes (Hybond N, Amersham) and UV-crosslinking (UV StratalinkerTM 1800 from Stratagene), followed by hybridization with the radioactively labeled probe. The prehybridization and hybridization steps were performed at 62°C in 7% SDS-phosphate buffer (1 mM EDTA, O.5 M NaHPO4 pH 7.2 and 7 % SDS), the washing steps at 62°C in 5% SDS-phosphate buffer (1 mM EDTA, 40 mM NaHPO4 pH 7.2, 5 % SDS). Filters were finally exposed to X-ray films.

### EXAMPLE 8: Subcloning of the genomic DNA.

The plasmid DNA (± 10 *µ*g; Qiagen purified) of positive clones, obtained after screening of the genomic DNA library, was sonicated (Vibra Cell VC500; Sonics & Materials, Danbury, CT) on ice in sonication buffer (1 M tetramethyl ammonium chloride, 2 mM EDTA, 50 mM Tris-HCl pH 7.6) for 30 seconds (50% duty cycle).

After precipitation with isopropanol, the DNA was blunted using 5 units each of T4- and Klenow-DNA polymerase (Boehringer) in T4 DNA polymerase buffer (50 mM Tris-HCl pH 8.5, 15 mM (NH4)2SO4 7 mM MgCl2, 0.1 mM EDTA, 10 mM β-mercaptoethanol; Boehringer), supplemented with 1 mM dNTP, at 37°C for 30 min. The fragments were size-fractionated by electrophoresis on a 1% agarose gel and the DNA of the portion of the gel comprising fragments from 800 to 1,200 bp was eluted and purified using the Geneclean method.

The blunted fragments were introduced into the dephosphorylated SmaI site of pUC18. Ligation was performed (molar ratio of vector/insert 1/3) using T4 DNA ligase (Pharmacia LKB Biotechnology) in blunt-end buffer (25 mM Tris-HCl pH 7.5, 5 mM MgCl2, 2.5% (w/v) PEG 8,000, 0.5 mM DTT, 0.4 mM ATP) at 23°C for 4 hours. The ligation mixes were transformed into E. coli MC1061 to generate two libraries of subclones.

### EXAMPLE 9: DNA preparation for Aspergillus transformation.

Plasmids were isolated by a modified protocol based on the 'cleared lysate' procedure of Kahn & al. (Meth. Enzymol., 1979, 68: 268) and purified by equilibrium centrifugation in a cesium chloride - ethidium bromide gradient.

A one liter culture (TB medium) was centrifuged in a GSA Sorvall rotor for 10 min at 5,000 rpm. The pellet was resuspended in 15 ml of lysing buffer (25% sucrose, 50 mM Tris-HCl pH 8, 20 mM EDTA) and 0.6 ml of a lysozyme suspension (10 mg lysozyme/ml H2O; Boehringer) was added.

After 30 min incubation on ice 15 ml of 2% Triton X-100 were mixed with the suspension and again incubated on ice for 30 min.

Then, the suspension was centrifuged in a SS34 Sorvall rotor for 30 min at 20,000 rpm. Cesium chloride (1.1 g cesium chloride/ml supernatant) as well as 1500 *µ*l of ethidium bromide ( 5 mg/ml) were added. This suspension was ultracentrifuged at 25°C and 55,000 rpm for 16 hours.

After isolation of the plasmid DNA band, the remaining ethidium bromide was removed by several extractions with an 1/1 volume of isobutanol (centrifugation at 10,000 rpm for 10 min) and the DNA was further precipitated (several times) with 0.6 volume of isopropanol, washed with 70 % ethanol and finally resuspended in 200 *µ*l of H2O.

### EXAMPLE 10: Transformation of Aspergillus nidulans.

The transformation of A. *nidulans* was based on the procedure of Yelton et al. (Proc. Nat. Acad. Sci. USA, 1984, 81:.1470) and included firstly the protoplasting of the mycelium by lytic enzymes (20 mg/g mycelium; Sigma Chemical, St. Louis, MO) and secondly the transformation itself using a polyethylene glycol treatment.

107 protoplasts were transformed with 1 *µ*g DNA of pSa123 selection plasmid and 19 *µ*g DNA of the plasmid of interest. The protoplasts were spread onto *Aspergillus* minimal medium plates [containing 1.2 M sorbitol, 1.5% Noble agar (Difco Laboratories, Detroit, MI) and biotin (1 *µ*g/ml) but no arginin] included in a top agar (same composition as the plates, but with 0.8% agar). The plates were incubated at 37°C until sporulating colonies appeared (± three days). Spores of these transformants were restreaked several times onto the same selection medium to obtain single colonies.

### EXAMPLE 11: Small scale genomic DNA preparation.

108 spores were inoculated into 20 ml of *Aspergillus* minimal medium supplemented with biotin (1 *µ*g/ml) and incubated overnight at 37°C and 240 rpm.

Genomic DNA was isolated using a modified protocol based on the procedure of Raeder and Broda (Lett. Appl. Microbiol., 1985, 1:.17). The mycelium was harvested by filtration using a folded filter (Schleicher & Schuell, Dassel, Germany) and washed with H₂O with 0.5 M EDTA pH 8 and finally with 100% ethanol. The mycelium pellet was dried under vacuum for several hours and then ground to powder under liquid nitrogen. 100 mg of mycelium were resuspended in 500 *µ*l of lysing buffer (200 mM Tris-HCl pH 8.5, 250 mM NaCl, 25 mM EDTA, 0.5 % SDS) and extracted twice with one volume of phenol/ chloroform/isoamylalcohol (25/24/1 - v/v/v) and once with chloroform/ isoamylalcohol (24/1 - v/v). The DNA was precipitated with 0.6 volume of isopropanol (after addition of 0.1 volume of 3 M potassium acetate pH 4.8), washed with 70% ethanol and resuspended in H₂O with RNAse A (50 *µ*g/ml; Sigma Chemical). After incubation at 37°C for 30 min, the DNA is stored at -20°C.

### EXAMPLE 12: Southern blot analysis.

10 *µ*g of *Aspergillus* genomic DNA of were digested with 120 units of Eco RI (New England Biolabs Inc., Beverly, MA) at 37°C overnight in the appropriate EcoRI buffer (total volume of 200 *µ*l). A 0.8 % agarose gel was run and stained with ethidium bromide.

After electrophoresis, the gel was incubated in 0.25 M HCl for 15 min. Transfer of the DNA fragments onto a Hybond N+ filter was performed using the 'alkali blotting' protocol of Amersham.

The filter was hybridized with the 1,150 bp DNA fragment described above, which was labeled with α32P-dCTP. The prehybridization and hybridization steps were performed at 68°C in 7% SDS-phosphate buffer (1 mM EDTA, 0.5 M NaHPO4 pH 7.2 and 7% SDS). The filter was washed twice with 5% SDS-phosphate buffer (1 mM EDTA, 40 mM NaHPO4 pH 7.2, 5% SDS) and once with 1% SDS-phosphate buffer (1 mM EDTA, 40 mM NaHPO4 pH 7.2, 1% SDS) at 68°C and finally exposed to X-ray film.

### EXAMPLE 13: Enzymatic assay

Enzymatic assay was performed in microtiter plates using N,N-dimethyl-p-phenylenediamine as substrate. 100 *µ*l of substrate solution (0.4 mg of N,N-dimethyl-p-phenylenediamine per ml of 40 mM sodium acetate pH 5.3) were added to 25 *µ*l sample at the appropriate dilution and incubated at room temperature protected from light. The optical density was measured at 550 nm.

The activity was expressed arbitrarily as ΔAbs/sec.

For tea color modification studies, the laccase activity was determined with 2,2'-azinobis(3-ethylbenzthiazoline-6-sulfonic acid (ABTS - Sigma-Aldrich Chemie, Germany) as substrate. After oxidation, its greenish-blue color was measured photometrically at 415 nm. Enzymatic assay were performed in microtiter plates using 75 *µ*l of 1.66 mM ABTS in 100 mM Citrate buffer pH 4 which were mixed with 25 *µ*l of supernatant and incubated during 10 min at 50°C.

1 laccase unit (LACU) is the amount of enzyme that catalyzes the conversion of 1 *µ*mole ABTS per minute in these conditions.

### EXAMPLE 14: Deglycosylation with PNGase F.

To a concentrated protein suspension (final volume of 90 *µ*l) 10 *µ*l of denaturation buffer (5% SDS, 10% β-mercaptoethanol) was added and the mix was boiled for 10 min at 100°C.

After addition of 12 *µ*l of NP40 (10 %) and 12 *µ*l of 0.5 M sodium phosphate pH 7.5, the sample was divided into two aliquots. 3000 units of PNGase F (peptide N-glycosidase F; New England Biolabs Inc.) were added to the first sample. The other one was used as negative control.

Incubation was performed overnight at 37°C. After addition of 2 x Laemmli buffer (Nature, 227:680(1970)) and boiling for 5 min, the samples were run on a 12.5% SDS-polyacrylamide gel.

The protein bands were visualized by Coomassie blue staining.

### EXAMPLE 15: Exchange of the Aspergillus ficuum DSM932 oxidase gene promoter by the gpdA promoter of A. nidulans.

The gene, encoding the oxidase protein, was isolated from the YCp50 SfiI/SfiI vector by a BamHI-SspI digestion and ligated to the plasmid pMa58 (Stanssens & al, 1989, Nucl. Ac. Res., 17: 4411) prepared in the following way: the pMa58 plasmid was digested with Hind III and the sticky ends were filled in with dNTP by T4 DNA polymerase and further digested with BamHI and PvuI. The resulting plasmid was termed pMa58Afic. A BspHI site was then created at the position of the ATG codon of the oxidase gene via site-specific mutagenesis, based on the method of Deng and Nickoloff (Anal. Biochem, 200: 81 (1992); TransformerTM Site-Directed Mutagenesis Kit of Clontech Inc., CA, USA), resulting in plasmid pMac58Aficm.

Via an XbaI digest on pMac58Aficm, followed by blunting the sticky ends with dNTP by T4 DNA polymerase, and a BspHI digest the oxidase gene was isolated and ligated into the pFGPDGLAT2 vector. The pFGPDLAT2 plasmid contains the glyceraldehyde -3-P dehydrogenase promoter of *Aspergillus nidulans* that allows a strong constitutive transcription of the genes located downstream of it (Punt & al, 1990, Gene, 93:.101; Punt & al, 1991, J. Biotechnol. 17:.19).

pFGPDGLAT2 was digested first with HindIII, treated with T4 DNA polymerase and finally digested with NcoI. The BspHI - XbaI fragment of pMac58Aficm was then ligated to this vector to generate the plasmid pFGPDAfic.

### EXAMPLE 16: PCR analysis of Aspergillus transformants.

Genomic DNA was isolated from the transformants as described. The oligonucleotides used for the PCR reaction were the following : 5' GAA GTG GAA AGG CTG GTG TGC (SEQ ID NO 51), corresponding to a partial sequence of the gpdA promoter, and 5' CAA CCC AGG TAC CGT ACT CC (SEQ ID NO 39), corresponding to a partial sequence of the oxidase gene.

50 ng of genomic DNA was suspended in 50 *µ*l of thermophilic buffer (50 mM KCl, 10 mM Tris-HCl pH 9.0, 0.1% Triton X-100; Promega Corporation), supplemented with 0.2 mM dNTP, 3 mM MgCl₂, 25 pmole of each primer and 1.5 units of Taq-DNA-polymerase (Promega Corporation). The temperature scheme for the amplification was as follows: After a 10-min denaturation at 95°C, a hot start at 80°C was used for the Taq-DNA-polymerase. 30 cycles of [95°C 30 sec, 65°C 30 sec, 72°C 2 min] were performed. The reactions were carried out in a Biometra "Trio-Thermoblock" thermocycler (Biometra). The PCR products were analyzed by electrophoresis on a 0.8% agarose gel.

### EXAMPLE 17: Purification of an enzyme with oxidase activity from Aspergillus ficuum DSM932.

### 1. Obtention of the desalted cell-free supernatant

A 10 l fermentation of the strain *Aspergillus ficuum* DSM932 was performed for 68 hours as described in example 2.

The supernatant of the culture was separated from the mycelium by centrifugation. It was desalted by passing through a 6 liters Sephadex G-25 column (Amersham Biosciences) equilibrated in 10 mM Tris-HCl, pH 7.0 buffer (buffer A). The same buffer was used to elute the proteins from the column. The final volume of the oxidase containing sample was 25 liters.

### 2. Chromatography on DEAE Macro Prep.

The sample of step 1 was applied on a 1l DEAE Macro Prep (Bio-Rad) column equilibrated with buffer A. Flow rate was 145 ml/min. After washing with the same buffer, the bound proteins were eluted by increasing stepwise the NaCl concentration in buffer A to give the following fractions: fraction 1 eluted with 1.5 l buffer A + 50 mM NaCl, fraction 2 eluted with 1.5 l buffer + 60 mM NaCl, fraction 3 eluted with 1.5 l buffer A + 110 mM NaCl, fraction 4 eluted with 1.5 l buffer l + 200 mM NaCl and fraction 5 eluted 1.8 l buffer A + 500 mM NaCl. Oxidase activity was detected in fraction 5.

### 3. Chromatography on S-Sepharose.

The buffer of 200 ml of fraction 5 from step 2 was exchanged to buffer B (15 mM ammonium acetate, pH 4.0) by passing through a Sepharose G-25 (Amersham Biosciences) column equilibrated in the same buffer.

The 260 ml eluted from G-25 column were applied on a 20 ml Hiload S-Sepharose HP 16/10 column (Amersham Biosciences) equilibrated in buffer B.

After washing with buffer B, proteins were eluted with a linear gradient of NaCl in buffer B (250 ml from 0 to 250 mM NaCl at 5 ml/min). 5 ml fractions were collected and tested for oxidase activity.

Active fractions (10 ml) were pooled and concentrated with Centriprep YM-30 centrifugal filter unit (Millipore). Buffer was exchanged to buffer C (50 mM MES - pH 5.5 with NaOH). The final volume was 400 *µ*l.

### 4. Chromatography on Resource Q.

200 *µ*l from step 3 were applied on a 1 ml Resource Q column (Amersham Biosciences) equilibrated in buffer C.

After washing with buffer C, proteins were eluted with a linear gradient of NaCl in buffer C (15 ml from 0 to 175 mM NaCl at 1 ml/min).

### 5. Chromatography on TSKG3000SW and TSKG2000SW.

Fractions with oxidase activity from step 4 (3.5ml) were concentrated by centrifugation to 400 *µ*l on a Centriprep YM-30 centrifugal filter unit (Millipore). Thereafter, 100 *µ*l were applied on TSKG3000SW and TSKG2000SW columns (Tosohaas) mounted in series and equilibrated in buffer D (20 sodium phosphate, 200 mM NaCl, pH 6.5). The proteins were eluted with buffer D at 0.5 ml/min. Active fractions were pooled (1.5 ml). 1 ml was desalted using a Microcon YM-10 centrifugal filter unit and concentrated to a volume of 200 *µ*l.

### 6. SDS-PAGE electrophoresis

The purified sample from step 5 was loaded on a 10-15% SDS polyacrylamide gel (PhastGel Gradient - 10-15; Amersham Biosciences) and run according to the recommendations of the manufacturer. One half of the gel was stained using the PhastGel Silver staining kit (Amersham Biosciences) and the other half was subjected to a zymogam analysis.

For this purpose the gel is incubated in a N,N-dimethyl-p-phenylenediamine solution (Sigma Chemicals; 0.4 mg/ml of 40mM sodium acetate pH 5.3) at room temperature. Proteins with oxidase activity appear black.

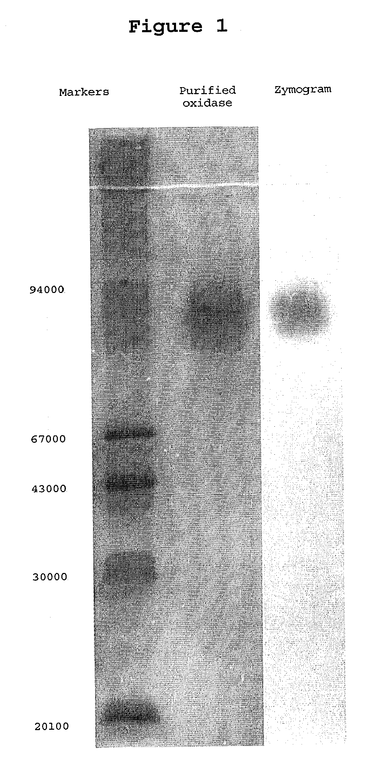

The results of this analysis are presented on figure 1. It shows that the enzyme with oxidase activity is pure and that it exhibits oxidase activity. The apparent molecular weight of the purified oxidase is about 90 kDa.

### EXAMPLE 18: Determination of the amino acid sequence of the enzyme with laccase activity

General procedures were followed to perform the N-terminal sequencing of the protein after electrophoresis on a 12% SDS-polyacrylamide gel and electroblotting on a PVDF Immobilon-P membrane (Millipore). An automatic 477A Protein Sequencer coupled to a HPLC 120A Analyser (Applied Biosystem) was used.

For the determination of the sequence of internal fragments, the protein was first digested on the membrane with trypsine. The resulting:peptides were separated by reverse phase chromatography on HPLC, and subjected to N-terminal sequencing as above.

The following sequences have been obtained:

- N-terminal: A V V Q F Q L D L T Y E D V S V A G X V X K A
I V L N G X I (SEQ ID NO 48)

- Internal peptide 1: S E D Q A G D Y T I R (SEQ ID NO 47)

- Internal peptide 2: A S Q Y X S Y I Y H S H T R (SEQ ID
NO 50)

### EXAMPLE 19: cloning of an oxidase encoding gene from Aspergillus ficuum DSM932

### Partial cloning of an oxidase gene from A. ficuum DSM932.

Based on known partial protein sequences encoded by the oxidase gene, the polymerase chain reaction (PCR) was used to isolate a DNA fragment, which has then be used as a probe to screen a genomic DNA library of A. *ficuum* DSM932. A set of degenerated primers (oligonucleotides) were synthesized based on the amino-terminal sequence and on the sequence of an internal peptide of the purified protein.

These nucleotide primers were:

- forward PCR-primer    5'  GTI GTI CAG TTT CAG YTI GAT YTI
  AC 3' (SEQ ID NO 37);

- reverse PCR-primer 3' CTY CTR GTY CGI CCI CTG ATG TGI
TA 5' (SEQ ID NO 38).

These two primers were used for PCR amplification on the genomic DNA of *A. ficuum* DSM932. The PCR reaction mixtures were analyzed directly by electrophoresis through a 1.3% agarose gel, followed by ethidium bromide staining.

Two weak DNA bands of ± 550 and 700 bp and one strong DNA band of 1,150 bp were generated.

Nucleotide sequence analysis revealed the relation between the 1,150 bp PCR product and the purified enzyme with oxidase activity.

For this, the PCR products were cloned in the pUC18 vector as described in example 5. Sequencing with the "universal" forward and reverse oligonucleotides primers (compatible with the insert-flanking regions of the vector) resulted in sequence determination of about 400 nucleotides on both sides of the insert. These nucleotide sequences were translated to peptide sequences. These peptides sequences included the sequence YEDVSVAGKVXXAIVLNG (SEQ ID NO 49), corresponding to a part of the amino terminal sequence determined in example 18 (from amino acid 11 to 28, SEQ ID NO 48) and the sequence ASQYXSYIYHSHTR (SEQ ID NO 50) corresponding to the sequence of the internal peptide 2 described in example 18.

### Cloning of the entire oxidase encoding gene of A. ficuum DSM932.

Using PCR amplification, groups of clones of the genomic DNA library, containing plasmids with the oxidase gene, were found.

The PCR reaction was performed on plasmid DNA (80 ng), extracted by the Birnboim procedure from 20 pools of the library, each of about 1,000 clones. The resulting PCR products were analyzed by electrophoresis through a 1.2% agarose gel.

Six groups of clones generated a PCR product of the expected size, 1, 150 bp, the same length as obtained from the original PCR amplification on the genomic DNA of *Aspergillus ficuum* DSM932. Three of these positive pools were further screened by colony hybridization using the radioactively labeled 1,150 bp DNA fragment as probe. Five positive signals were obtained after autoradiography detection.

The positive clones were further purified by a new cycle of colony hybridization, and by PCR amplification of the 1150 bp fragment from the purified plasmids. This finally resulted in the isolation of two single positive clones.

The DNA of the positive clones was analyzed by SfiI digestion, resulting in the release of the genomic insert from the YCp50 vector.

One clone showed after electrophoresis on a 0.8 % agarose gel a genomic insert of a length of 11,000 bp and the other one an insert of more than 12,000 bp. Both inserts contained an internal SfiI cleavage site. The plasmid with the 12,000 bp insert was termed pAFLAC and has been deposited as an *E. coli* MC1061 transformant at the LMBP collection with reference number LMBP4366.

BELDEM S.A., who has registered office at B-5300 Andenne (Belgium) Rue Bourrie, 12, has made on 11 May 2001 (under the expert solution) of the microorganism Escherichia coli MC1061 (pAFLAC) according to the invention, at the BCCM/LMBP Culture Collection (Laboratorium voor Moleculaire Biologie, Universiteit Gent, 'Fiers-Schell-Van Montagu' building, Technologiepark 927, B-9052 Gent-Zwijnaarde, Belgium). This deposit has received the accession number LMBP 4366.

### Sequence analysis of the A. ficuum DSM932 oxidase gene.

Genomic DNA inserts from pAFLAC were sheared and subcloned in the pUC18 plasmid vector. The ligation mixes were transformed into E. coli MC1061 to obtain two libraries of subclones. Subclones, containing part of the desired gene, were selected after colony hybridization using the radioactively labeled 1,150 bp PCR fragment as probe.

Purified (Qiagen) plasmid DNA of 12 positive subclones was further sequenced and the DNA sequences obtained were analyzed.

This random sequencing gave the nucleotide sequence of the first 1,100 bp of the gene as well as 500 bp of the promoter region. To obtain the entire gene sequence, primer walking was performed to sequence the 3' end of the gene. Remaining ambiguities were resolved using custom primers. Only one of the two positive genomic clones (the one with the genomic DNA insert of more than 12,000 bp) appeared to contain the entire gene.

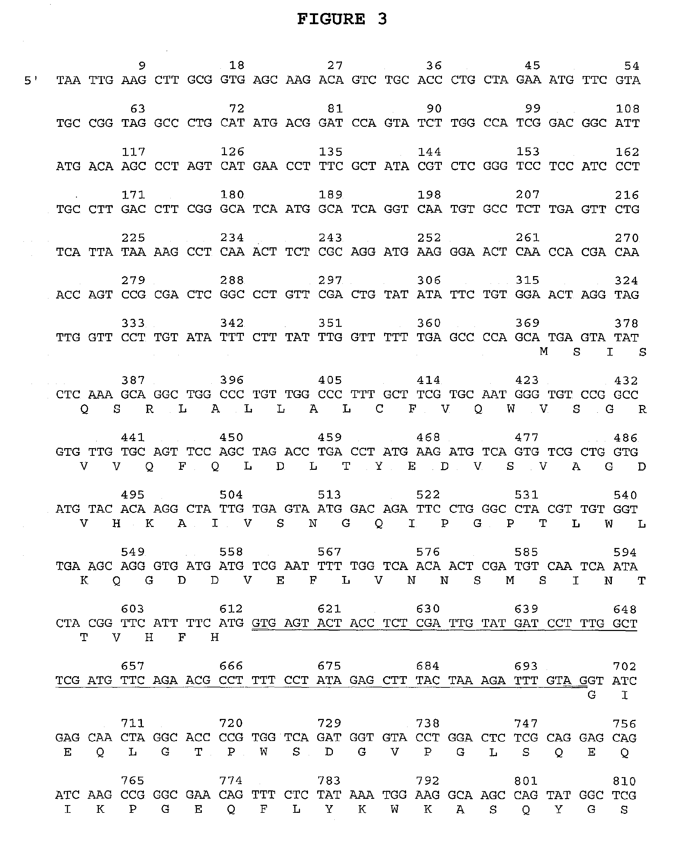

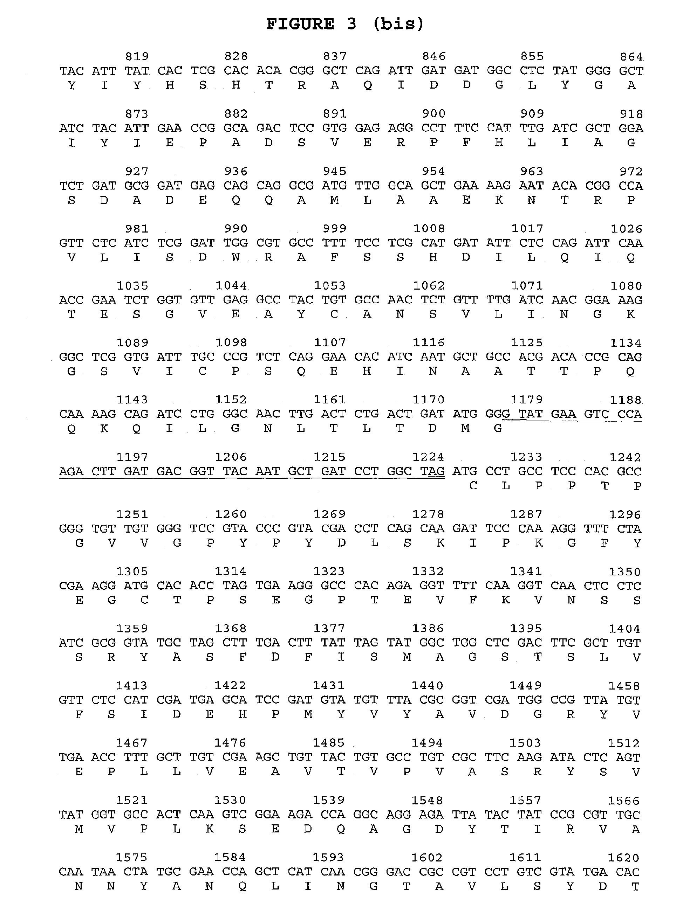

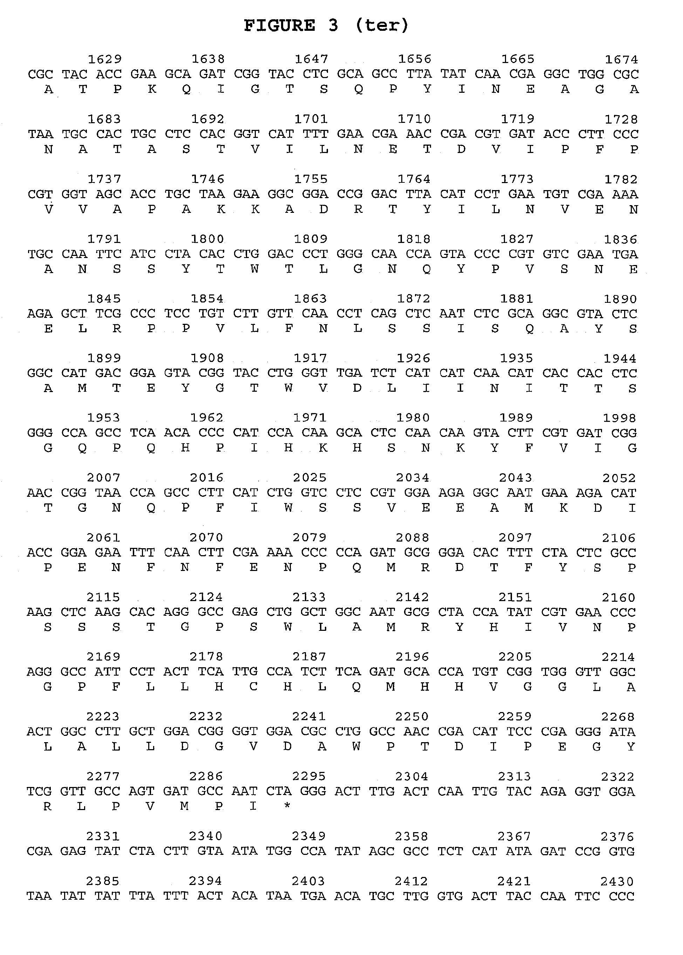

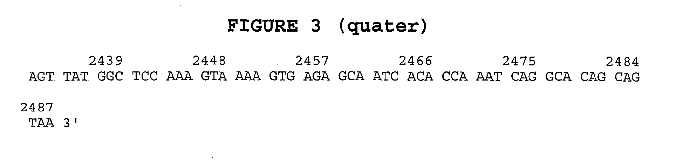

The nucleotide sequence and the corresponding amino-acid sequence (SEQ ID NO 1 and SEQ ID NO 40) are shown in figure 3. The gene is 1,923 base pairs. It contains two introns of respectively 85 (starting at base pair 245) and 49 (starting at base pair 808) base pairs and encodes an open reading frame of 596 amino acids with a calculated molecular weight of 65,476 Da.

A search for similarities between the sequence obtained and sequences in databases was performed using the BLAST program (Basic Local Alignment Search Tool; Altschul & al, 1990, J. Mol. Biol., 215, p.403). The closest homologous gene product was a hypothetical protein from *Aspergillus nidulans* FGSC A4 (AN 0878.2 - accession number EAA65907). The overall homology was 56.7% as found by analysis with the Clustalw program using the default parameters (http://www.ebi.ac.uk.clustalw).

### EXAMPLE 20: Heterologous expression of the oxidase gene.

The fungal strain *Aspergillus nidulans* 2024, which is auxotrophic for biotin and arginin, was chosen as host strain for the expression of the isolated *Aspergillus ficuum* oxidase gene.

Cotransformation of the positive clone (YCp50 vector with the genomic DNA insert of more than 12,000 bp) with the selection plasmid pSal23 (John & al, 1984, Enzyme Microbiol. Technol., 6:.386), containing the argB-gene, was performed.

Whether the resulting transformants (obtained on plates without arginin) had also integrated the positive clone, was checked using PCR amplification and Southern blot analysis.

Genomic DNA was isolated from cultures of the transformants. This genomic DNA was submitted to a PCR amplification reaction. DNA of *A. ficuum* DSM932 and of the untransformed A. *nidulans* 2024 were used as positive and negative controls, respectively.

The PCR products were analyzed by electrophoresis on a 1.2% agarose gel with ethidium bromide staining.

The PCR reaction with the DNA of *A. ficuum* DSM932 or the DNA from some transformants generated as expected a 1,150 bp DNA fragment, indicating the *A. ficuum* gene integration in the genome of these transformants. The PCR reaction on the untransformed *A. nidulans* gave no amplification signal. Southern blot analysis confirmed these results.

The cotransformants showed one or more signals on the blot, indicating one or more integrated *A. ficuum* gene copies.

A cotransformation efficiency of 60 % was obtained.

Another phenomenon, observed on the positive transformants, was the change of the spore color. Non-transformed and pSa123-transformed *A. nidulans* colonies showed dark green spores, while transformants with the oxidase gene integrated gave yellow spores.

### Analysis of Aspergillus nidulans transformants.

The transformants were checked whether expression of the integrated *A. ficuum* gene was established and whether the synthesized protein was enzymatically active.

Transformants were grown in *Aspergillus* minimal medium. *A. ficuum* was used as positive control, and the untransformed and pSa123-transformed *A. nidulans* as negative controls.

The proteins secreted in the medium were analyzed by electrophoresis on a 12.5% SDS-polyacrylamide gel. The culture medium samples were previously concentrated by deoxycholate-trichloroacetic acid precipitation. Proteins were stained on the gel with Coomassie blue. A very large protein band of 85 kDa, with a size close to the size of the *A. ficuum* oxidase, appeared only in the samples of the transformants of *A. nidulans.* The protein secreted by *A. nidulans* is about 5 kDa smaller than the one secreted by *A. ficuum.* This is due to differences in the glycosylation patterns, as shown below.

These results show that the isolated *A. ficuum* oxidase gene was expressed in *A. nidulans* under the control of its own promoter. More surprinsingly, the expression level was even higher in *A. nidulans* transformants than in the wild-type *A. ficuum* DSM932 strain.

Zymogram was performed to study the enzymatic activity of the expressed protein. Proteins in the culture medium were concentrated and loaded on a SDS-polyacrylamide gel under semi-denaturating conditions (absence of β-mercaptoethanol; no boiling of the samples before loading). After electrophoresis, the gel was incubated in a N,N-dimethyl-p-phenylenediamine substrate solution. The observed protein bands of *A. ficuum* DSM932 and of the *A. nidulans* transformants were firstly pink-colored and became black after a longer incubation time, confirming that the protein could oxidize the substrate and was enzymatically active. The same results were also obtained in solution in an oxidative assay with the same substrate N,N-dimethyl-p-phenylenediamine. The medium of the untransformed *A. nidulans* showed no activity at all.

The results obtained above demonstrated that the integrated *A. ficuum* gene in the *A. nidulans* transformants code for an enzymatically active oxidase. Some transformants showed more activity than the original *A. ficuum* strain, due to a higher gene expression.

### EXAMPLE 21: Glycosylation of the enzyme with oxidase activity.

*A. ficuum* DSM932 and one *A. nidulans* transformants were grown in *Aspergillus* minimal medium. The secreted proteins in the medium were concentrated and further submitted to a PNGase F treatment. Then, the deglycosylated samples were loaded on a polyacrylamide gel and a Coomassie blue staining was performed.

A clear shift of the protein pattern was visible. Deglycosylation with PNGase F decreased the molecular weight of the two oxidases to about 70 kDa. This experiment showed that the observed 5 kDa difference in size of the non-deglycosylated oxidases of *A. ficuum* and *A. nidulans* transformant was indeed due to differences in glycosylation, since deglycosylation resulted into proteins of the same size.

### EXAMPLE 22: Expression of the enzyme with oxidase activity in various Aspergillus species under the control of the gpdA promoter.

The promoter of the isolated *A. ficuum* oxidase gene was replaced by the constitutive gpdA (glyceraldehyde-3-phosphate dehydrogenase A) promoter of *A. nidulans,* known as a strong fungal promoter (Deng & al, 1992, Anal. Biochem., 200: p.81).

This plasmid was transformed into A. *nidulans* **2024,** *A. niger* N402 and A. *ficuum* DSM932.

The transformation procedure was based on the cotransformation with the pSa123 (arginine selection).

To transform A. *niger* and A. *ficuum* another selection procedure was used, based on the acetamidase encoding gene (Kelly & al, 1985, EMBO J., 4: 475). In these cases, the plasmid p3SR2, containing the amdS gene (encoding the acetamidase) of A. *nidulans,* was used for the cotransformation, resulting in colonies which could grow on acetamide as sole nitrogen source.

The transformants obtained (146 for *A. nidulans,* 89 for *A. niger* and 326 for *A. ficuum)* were analyzed by PCR amplification with a set of two primers. The upstream primer hybridized with a sequence in the gpdA promoter and the downstream primer with a sequence in the oxidase gene. A DNA band of 2281 bp (corresponding to the expected size between the sequences of the oligonucleotide primers in the sequences of the gpdA promoter and the oxidase gene) was detected in the PCR mix of some transformants. This demonstrated that these transformants were cotransformed and thus had integrated not only the selection plasmid but also the plasmid pFGPDAfic.

These cotransformants of *A. niger* and *A. ficuum* exhibited the same spores color change, as mentioned above for the *A. nidulans* transformants (grey to light brown spores instead of black - example 4).

The transformants were further analyzed by SDS-polyacrylamide gel electrophoresis and by the enzymatic assay with the substrate N,N-dimethyl-p-phenylenediamine. The transformants were grown in *Aspergillus* minimal medium (5 ml in a 50 ml plastic tube) for three days and the culture supernatant was submitted to the enzymatic assay and to electrophoresis.

The best *A. nidulans* transformants showed an expression level 5.5 times higher than the expression obtained with the original oxidase promoter in *A. nidulans.*

This observation showed that promoter exchange resulted in a higher expression level of the oxidase in *A. nidulans.*

The best transformants of *A. ficuum* exhibited also a 4-fold increase in comparison with *A. nidulans,* which was transformed with the oxidase, controlled by its own promoter.

The *A. niger* transformants secreted about the same amount.

### EXAMPLE 23: characterization of the oxidase

### 1. Obtention of an oxidase sample

One transformant of *Aspergillus ficuum* obtained in the example 22 was selected for further experiments. A 10 1 fermentation was performed as described in example 2.

The supernatant of the fermentation was desalted in the same conditions as described in example 17 and concentrated by ultrafiltration.

### 2. Determination of the optimum pH

The optimum pH of the oxidase was determined by assaying its activity at various pHs using a sodium phosphate-citrate buffer. The results of this experiment are shown in figure 3.

The optimum pH lies around pH 5.5.

### EXAMPLE 24: Tea color modification by laccase

30 ml of a green tea solution, made by preincubation of commercial tea (Pickwick Green Tea, Douwe Egberts N.V., Belgium) in warm water (1g per 100 ml) during 15 min, were stirred with various amounts of *Aspergillus ficuum* oxidase (0,1 and 10 U per ml of green tea) at 40°C.

After 1 hour, tea solutions incubated with oxidase became red-brown, and their absorbance spectra were recorded between 350 and 680 nm and compared with that of the control without enzyme.

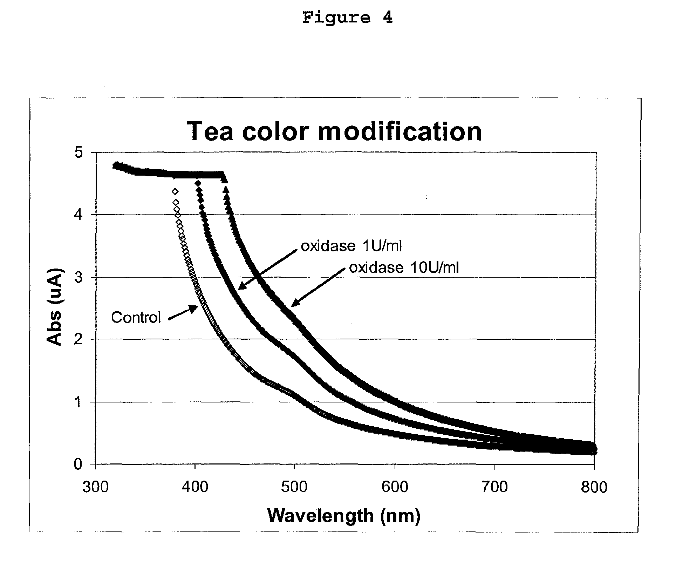

The results on Figure 4 clearly show that the use of the oxidase of *Aspergillus ficuum* enhance the color of the tea solution as compared with an untreated sample.

### EXAMPLE 25: Effect of the oxidase in baking

Baking trials were performed to demonstrate the positive effect of the oxidase of the present invention in baking. The positive effect was evaluated by the increase in bread volume as compared to a reference, which does not contain this enzyme.

The oxidase from example 23 was evaluated in mini baking tests consisting of preparing dough with 100g of flour.

The procedure described is well established and it will be readily apparent to a person skilled in the art that the same results may be obtained by using other protocols or equipments from other suppliers.

The ingredients used are listed in table 1 below:

**Table 1**

| Ingredients (g) | RECIPE |
|---|---|
| Flour (Surbi -Molens van Deinze) | 100 |
| Water | 57 |
| Instant yeast (Bruggeman-Belgium) | 2 |
| Sodium chloride | 1.5 |
| Dextrose | 6 |
| Fat (Solix (Puratos - Belgium) 80% / Soy oil 20%) | 3 |
| oxidase | see Table 2 |

The ingredients were mixed for 3.5 min in a National mixer. 150 g dough pieces were weighed and rested for 20 min at 25°C in plastic boxes.

The doughs were reworked and rested for a further 20 min. The final proofing time was 60 min at 36°C. The dough pieces were then baked at 220°C for 24 min.

The volume of the bread was measured using the commonly used rapeseed displacement method.

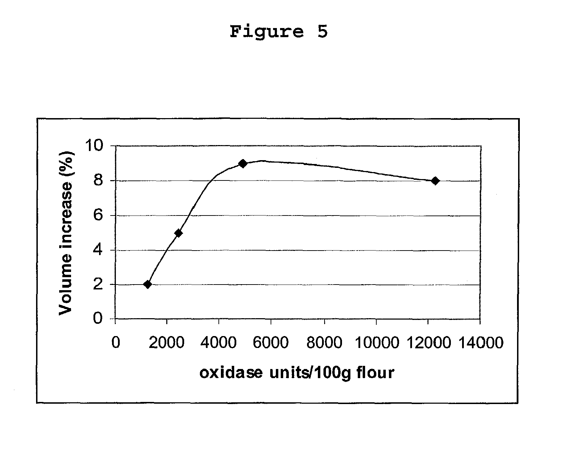

The results of the baking trials with the oxidase are presented in table 2 below and on figure 5:

**Table 2**

| Oxidase units /100 g flour(*) | Rolls volume increase (%) compared to control without oxidase |
|---|---|
| 1225 | 2 |
| 2450 | 5 |
| 4900 | 9 |
| 12250 | 8 |

| | |
|---|---|
| (*) See example 13 for the enzymatic assay | |

The above results show that the oxidase of the present invention has a positive effect on the volume of bread.

### EXAMPLE 26: Effect of the oxidase on rye flour dough stickyness

A series of doughs was prepared by mixing the following ingredients in a Farinograph mixer at 30°C for 2min.

**Table 3**

| | |
|---|---|
| Rye flour Werhahn | 200g |
| Wheat flour Duo Ceres | 20g |
| Water (tap at 37°C) | 125g |
| Salt solution (100g NaCl/1000 ml water) | 50 ml |
| Basic improver RB14120 (Puratos)* | 8g |
| Oxidase | see Table 4 |

| | |
|---|---|
| * The basic bread improver RB14120 contains alpha-amylase, xylanase, ascorbic acid and citric acid. | |

The dough stickyness was measured 5 min after mixing using a Stable Micro Systems TA-XT2i texture analyzer, equipped with a Chen-Hoseney dough stickyness cel.

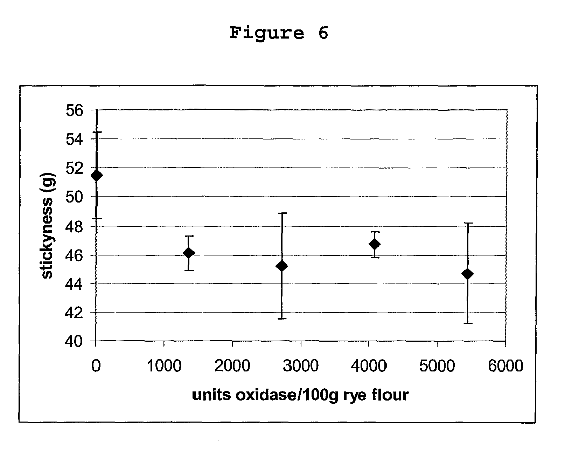

The results are presented on table 4 and on figure 6.

**Table 4**

| composition | Stickyness (g) | Standard deviation (g) |
|---|---|---|
| Reference | 51.5 | 3.0 |
| 1360u/100g Rye flour | 46.1 | 1.2 |
| 2720u/100g Rye flour | 45.2 | 3.7 |
| 4080u/100g Rye flour | 46.7 | 0.9 |
| 5440u/100g Rye flour | 44.7 | 3.5 |

From these data, it can be seen that the dough stickyness is significantly reduced by the addition of the oxidase according to the present invention.

### Example 27: effect of the oxidase on rye flour dough consistency.

The same doughs as described in example 10 were prepared.

The dough consistency was measured using a Physica UDS 200 Rheometer with the folowing parameters:
- oscillating mode
- temperature = 30°C
- plate-plate system ⌀=30mm, d=2mm
- frequency sweep: γ=0.02%, F increases linearly from 1 to 50Hz.

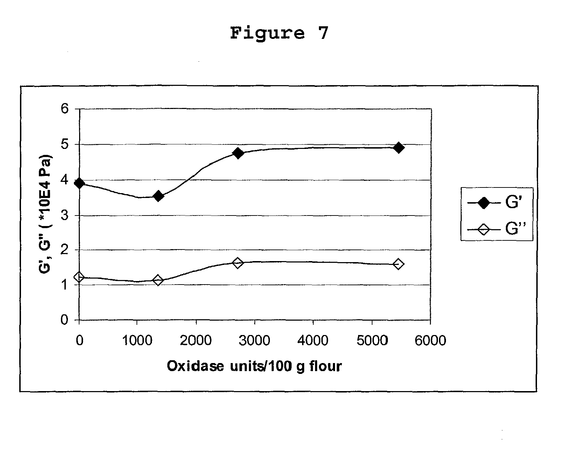

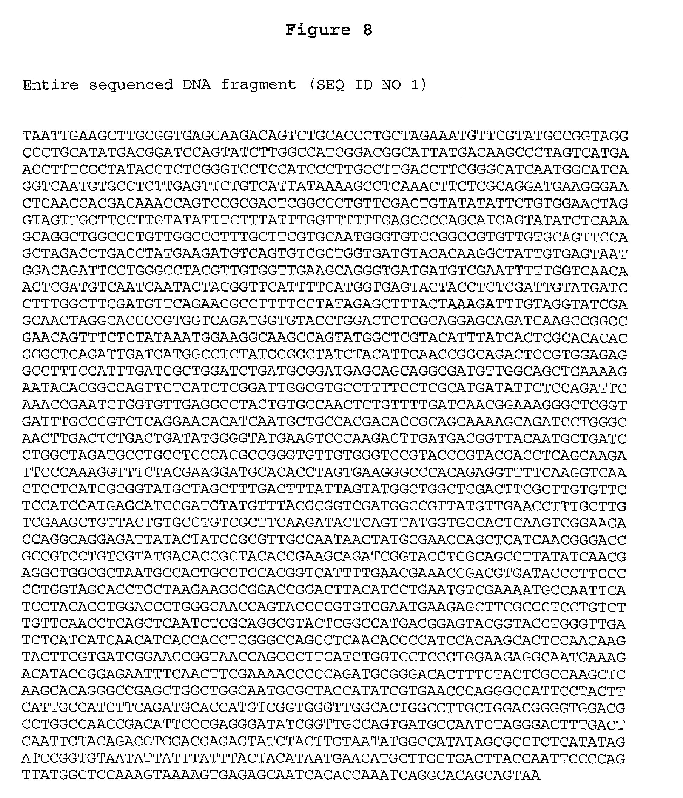

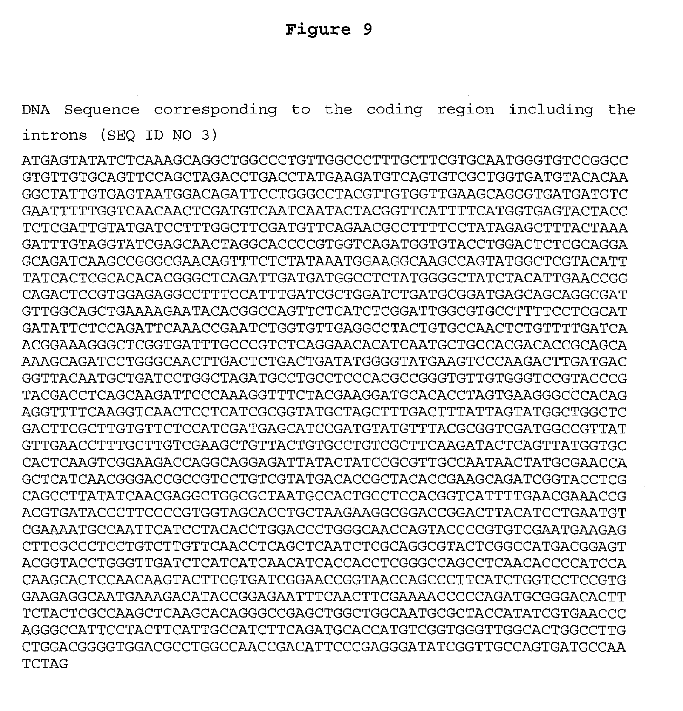

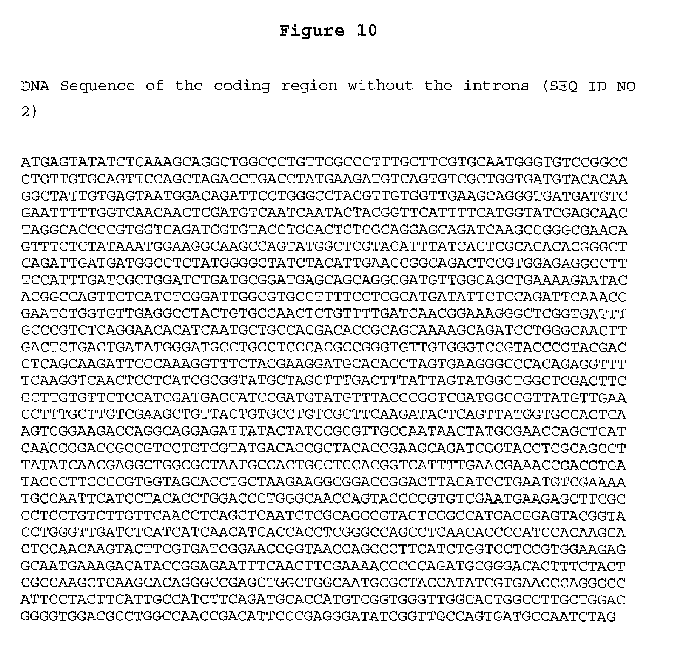

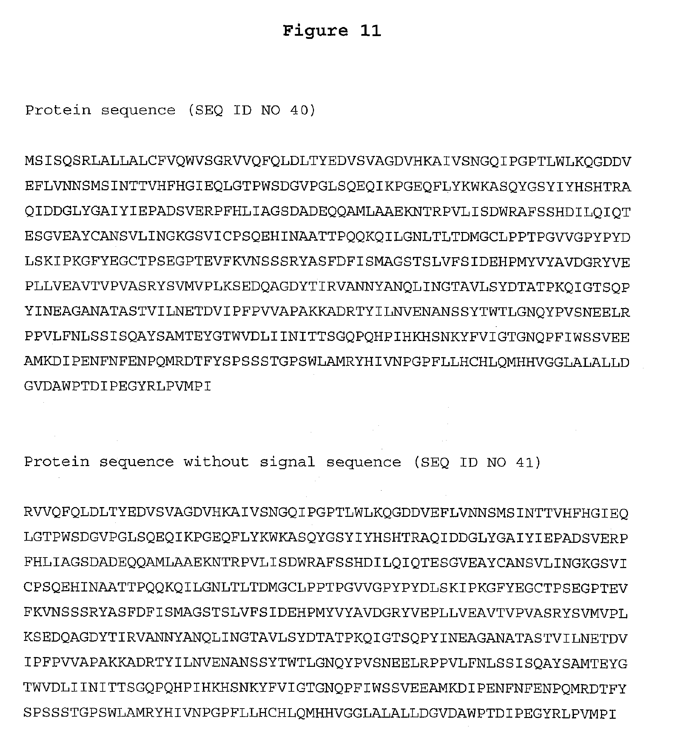

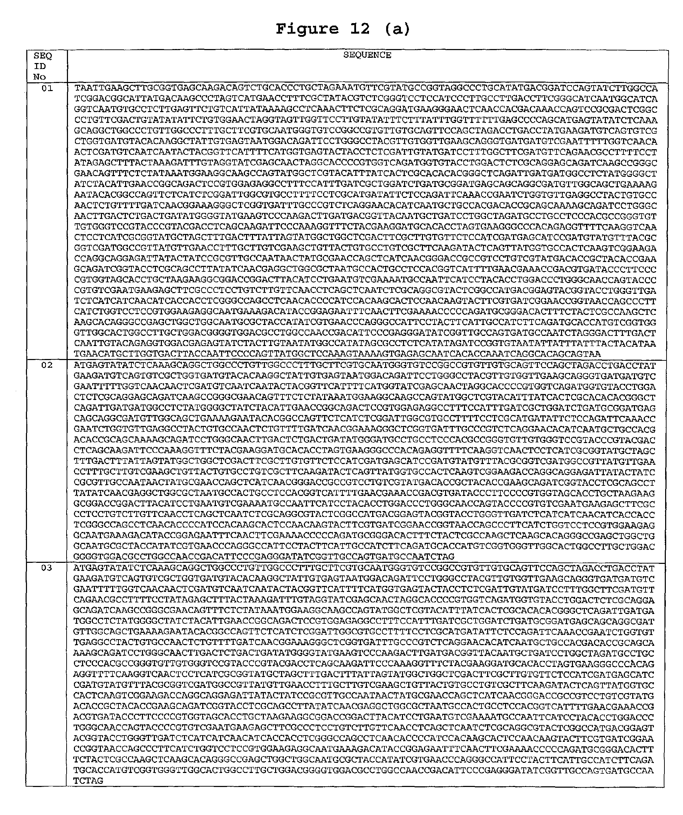

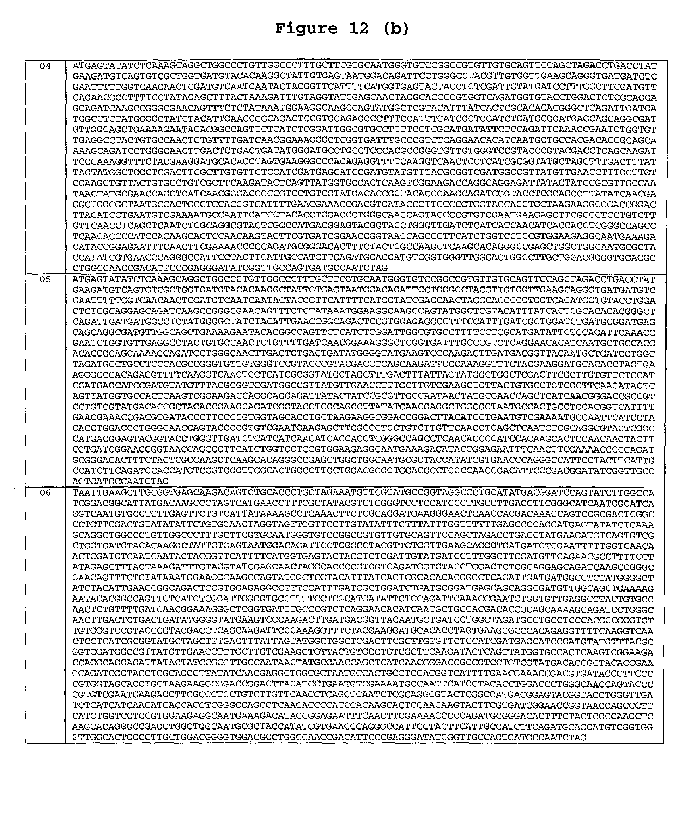

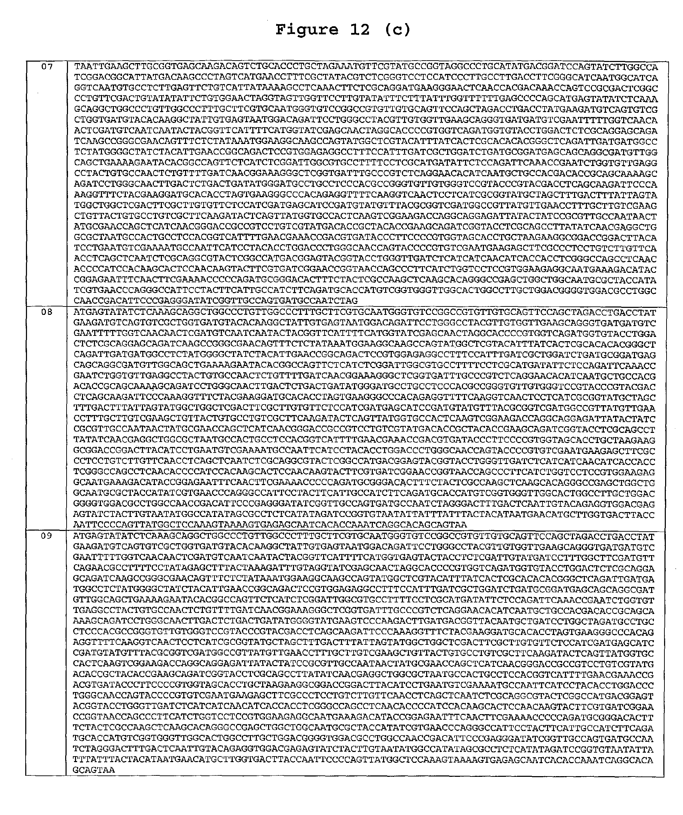

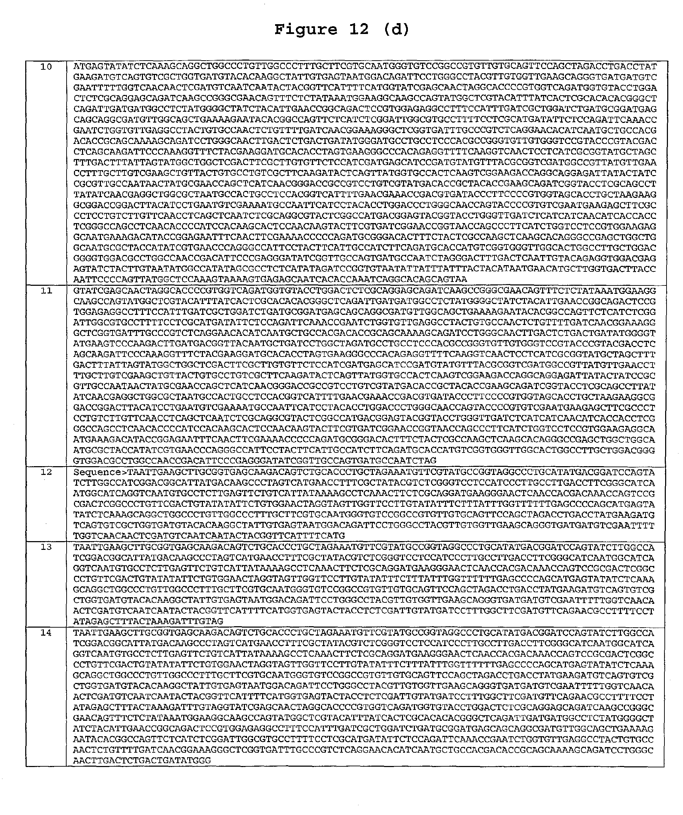

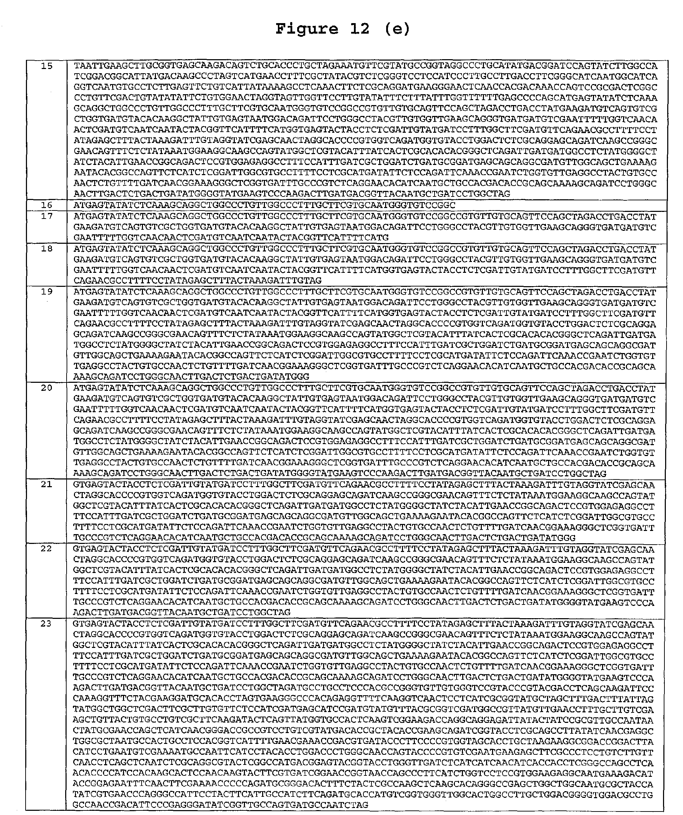

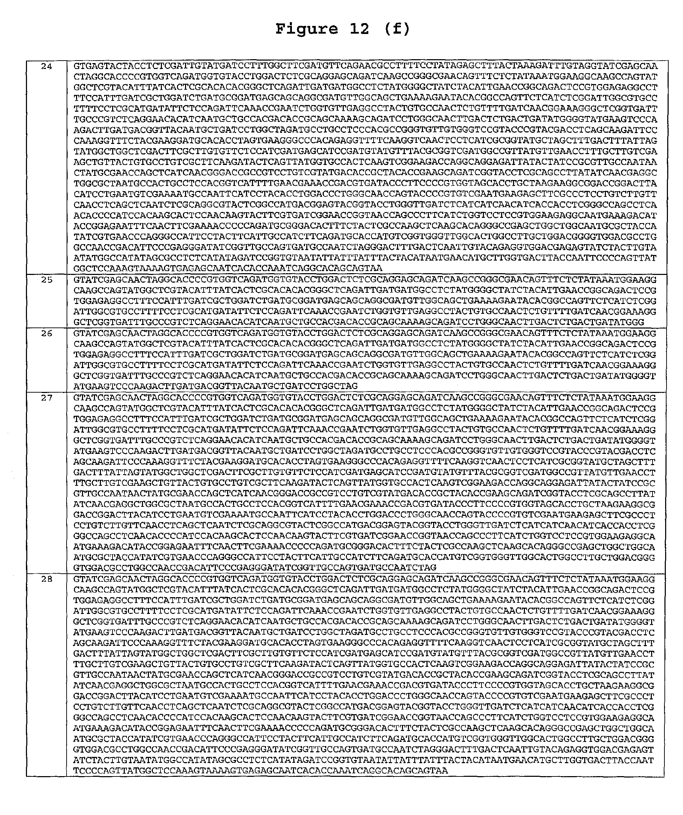

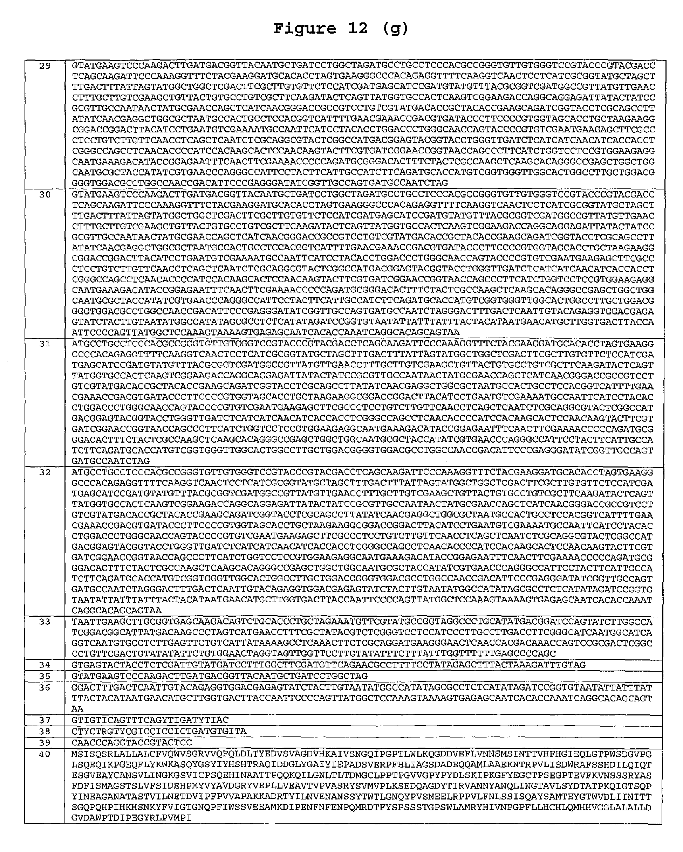

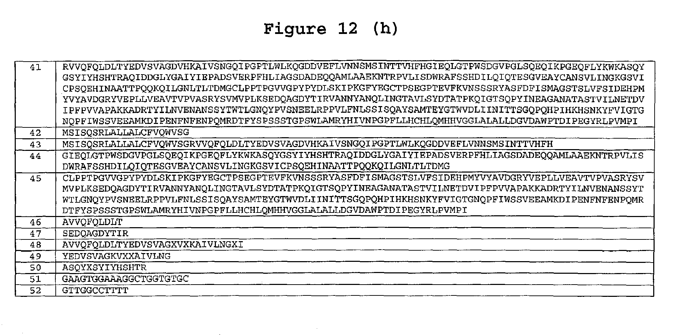

The results obtained at 10 Hz are presented on Table 5 and figure 7.

**Table 5:**

| composition | G' (*10^{E}4) | G" (*10^{E}4) |
|---|---|---|
| Reference | 3.88 | 1.23 |
| 1360u/100g Rye flour | 3.52 | 1.14 |
| 2720u/100g Rye flour | 4.73 | 1.63 |
| 5440u/100g Rye flour | 4.90 | 1.60 |

These data show that the dough consistency is significantly increased by the addition of the oxidase of the present invention. Moreover the dough becomes stiffer and permits a better handling and shaping of the dough.

## Claims

1. An isolated oxidase polypeptide comprising or consisting of:
- an amino acid sequence of any of SEQ ID NO 40 to 50,
- a fragment of at least 100 amino acids of SEQ ID NO 40, 41 or 45, or
- an amino acid sequence presenting at least 60% identity with the amino acid sequence of any of SEQ ID 40 to 50, or with any fragments of at least 100 amino acids of said SEQ ID NO 40, 41 or 45.

2. An isolated nucleic acid molecule encoding a polypeptide according to claim 1.

3. An isolated nucleic acid molecule comprising:
- a nucleotide sequence of any of SEQ ID NO 1 to 39, its complementary form or RNA form,
- a nucleotide sequence having at least 60% identity with any of SEQ ID NO 1 to 39, or with the complementary form or RNA form thereof,
- a fragment of any of SEQ ID NO 1 to 11 or of any of their homologues, or of any of their complementary form or RNA form, wherein said fragment encodes a protein having an oxidase activity, or
- a fragment of at least 15 nucleotides of any of SEQ ID NO 1 to 39 or of any of their homologues, or of any of their complementary form or RNA form.

4. An isolated nucleic acid molecule according to claim 3 wherein said fragments of any of SEQ ID NO 1 to 11 or of their homologues, or of any of their complementary form or RNA form, encoding a protein having an oxidase activity, consist of at least 200 nucleotides.

5. A vector comprising a nucleic acid molecule according to any of claims 2 to 4.

6. A vector according to claim 5 wherein said nucleic acid molecule according to any of claims 2 to 4 is operatively linked to one or more regulatory sequences.

7. A vector according to claim 5 or 6 wherein said regulatory sequence is the gpdA promoter of *Aspergillus nidulans.*

8. A transformed host cell containing a nucleic acid molecule according to any of claims 2 to 4.

9. A transformed host cell containing a vector according to any of claims 5 to 7.

10. A fusion protein comprising an oxidase polypeptide according to claim 1.

11. A bread improving composition comprising an oxidase polypeptide according to claim 1.

12. Use of an oxidase polypeptide according to claim 1 in a baking process or as color enhancer.
